# EUROPEAN PATENT APPLICATION

(11) **EP 2 614 709 A1**
(43) Date of publication of application: **17.07.2013**
(21) Application number: 13163405.7
(22) Date of filing: 15.07.2006
(51) Int. Cl.: A01K 67/00, A01K 67/027, A61K 38/00

(54) **Small animal model for HCV replication**

(30) Priority: 18.07.2005 US 700475 P; 23.02.2006 US 776640 P
(62) Divisional of application: 06787397.6
(71) Applicant: Novartis AG, 4056 Basel (CH)
(72) Inventor: Weiner, Amy, Emeryville, CA 94662-8097 (US); Aukerman, Sharon Lea, Emeryville, CA 94662-8097 (US); Mendel, Dirk, Emeryville, CA 94662-8097 (US); Zhu, Qing, Emeryville, CA 94662-8097 (US)
(74) Representative: Marshall, Cameron John

(57) **Abstract**

An animal model for HCV replication and/or production of virus or virus like particles is provided. The invention utilizes an HCV replicon present in a cell to deliver HCV nucleic acid and replicate and express HCV proteins in an animal model comprising an animal that has been immunocompromised. The invention further provides a method of treatment or prevention of HCV in a mammal which comprises administering to the mammal a combination which comprises an immunomodulatory compound and another antiviral agent. Also provided are cell lines showing a decreased sensitivity to interferon alpha or some other immunomodulator and methods of making or isolating such cell lines.

## Description

### BACKGROUND

Hepatitis C virus (HCV) infects only humans and chimpanzees. As a result, the HCV field suffers from not having robust small animal models of HCV infection and/or replication. Existing or proposed models of HCV infection and replication rely on complicated techniques and/or on the use of naturally occurring HCV infected human innocula. Some existing models use implanted naturally infected HCV cells or cells that are infected by HCV *ex vivo* or *in vivo* using human sera. Existing models also rely on the use of mice that have been genetically mutated to damage their livers and/or may use viral vectors to deliver HCV genes, which complicates the biology of the host. For example, in one approach, primary human hepatocytes (PHH) are implanted into the livers of liver-damaged (*Alb-uPA* SCID/*bg*) mice. Once these PHH are engrafted, the mice can then be infected (*in vivo*) with human sera containing HCV. HCV will then infect these human hepatocytes. Alternatively, PHH infected *in vivo* as a result of natural infections in the donor can be implanted into mice livers. In another approach, human hepatocellular carcinoma cells (HCC) are incubated with human plasma containing HCV *ex vivo* prior to being implanted into the livers of athymic nude mice. Also, it has been proposed to transform liver-damaged mice that are deficient in an essential hepatic protein with an expression system that includes control sequences operably linked to an HCV replicon and to a nucleotide sequence encoding at least one protein that overcomes the defect. Additionally, using adenoviral vectors to deliver HCV proteins such as protease has been described. Infectious models also include infecting pieces of liver *ex vivo* and implanting the infected liver into a mouse (XTL).

A disadvantage of these systems is that they are laborious, require special expertise to isolate and transplant fresh human hepatocytes and/or require transgenic animals (with high mortality rates), require infectious viral innocula and/or employ viral vectors that significantly effect cell biology of the host.

### SUMMARY OF THE INVENTION

The invention provides an animal that can be used in an animal model for HCV replication and/or production of virus or virus like particles. The invention utilizes an HCV replicon present in a cell to deliver HCV nucleic acid and replicate and express HCV proteins in an animal model comprising an animal that has been immunocompromised. The replicon further comprises a reporter gene and can be a genomic or subgenomic replicon.

The animal comprises a cell or cells containing an HCV replicon that is introduced into an animal that is immunocompromised. The cell or cells either already contain an HCV replicon (infected cells) or are transfected *in vitro* with an HCV replicon (transfected cells) that supports replication of HCV RNA and expression of viral proteins. Alternatively, the cells can be transfected in vivo in the animal with replicon RNA. The cells may also express additional proteins. The replicon further comprises a reporter gene to monitor replication and or expression of the replicon, or to monitor virus or virus like particle production from the replicon. After introduction of cells containing the replicon into an animal, replication and or expression of the HCV replicon may be measured *in vivo* or *in vitro.* Virus or virus like particle production can be monitored by examining the blood of the animal for virus or virus like particles. According to various embodiments, human-derived cells that are adapted for growth and replicon stability in the animal are used. In one embodiment, the animal is a small animal as described herein.

In another embodiment, the invention provides a combination of two or more antiviral agents and a pharmaceutical composition comprising two or more antiviral agents.

In another embodiment, the invention provides a method of treatment or prevention of HCV in a mammal which comprises administering to the mammal a combination which comprises an immunomodulatory compound and another antiviral agent. In one embodiment, the antiviral agent is an anti Hepatitis C virus agent that can be selected from the group including inhibitors of viral enzymes such as proteases, helicases, GTPases, polymerase or inhibitors of IRES and small molecules such as siRNA or antisense, inhibitors of HCV biology as for example, anti-cd81 compounds and the like and other known immnuomodulators as discusses further herein.

In another embodiment, the invention provides cell lines harboring genomic or subgenomic replicons that are derived according to the methods provided in the Example set forth below. In some embodiments, the cell lines show a decreased sensitivity to interferon or some other jimmunomodulator after having been passaged one or more times in an animal as described herein. In one embodiment, the invention provides an animal useful for determining the replication, expression or production of HCV virus or virus-like particles from an HCV replicon, the animal comprising: a cell containing an HCV replicon comprising a reporter gene wherein the animal is immunocompromised.

The cell containing the HCV replicon is selected from the group consisting of: primary hepatocytes; stem cells; kidney cells; liver cells; bone marrow-derived cells; hepatomas; hepatoblastomas and can be a human cell line and can be an immortalized cell line. In a preferred embodiment, the animal is a small animal and can be a rodent.

The invention also provides a method of identifying a compound that reduces or inhibits HCV replication comprising: a)providing an immuno-compromised animal according to any one of claims 1 to 45;b)administering a candidate compound to the animal; and c)determining the level of activity of the reporter gene contained within the replicon in the animal as compared to the level of activity of the reporter gene contained within the replicon in an animal as provided in step a) that is not contacted with the compound; wherein a compound that reduces or inhibits the level of activity of the reporter gene contained within the replicon in the animal contacted with the compound is a compound that inhibits or reduces HCV replication.

The invention also provides a method of identifying a compound that reduces or inhibits HCV particle production comprising: a) providing an immuno-compromised animal b) administering a compound to the animal; wherein the compound does not reduce or inhibit replicon replication or expression and c)determining the level of activity of the reporter gene contained within the replicon in the animal as compared to the level of activity of the reporter gene contained within the replicon in an animal as provided in step a) that is not contacted with the compound; wherein a compound that reduces or inhibits the level of activity of the reporter gene contained within the replicon in the animal contacted with the compound is a compound that inhibits or reduces HCV particle production.

The invention also provides a pharmaceutical composition comprising a therapeutically effective amount of a first antiviral agent and a therapeutically effective amount of a second antiviral agent.

The invention also provides a method for the treatment or prevention of a HCV infection in a mammal by administering to the mammal an anti-HCV effective amount of the pharmaceutical composition as described herein.

The invention further provides a method for establishing a cell line and cell lines as described in any of the General Methods described herein and the specific Examples. In one embodiment, the invention provides a cell line less sensitive to an immunomodulatory agent after passage through an animal of the invention as described herein.

In one embodiment the immunomodulatory compound is interferon alpha 2B.

In another embodiment, the invention provides a tumor cell line having a decreased sensitivity to interferon-α derived by the following process: implanting tumor cells containing an HCV replicon comprising a reporter gene (which on one embodiment is bioluminescent) into an immunocompromised mouse, wherein the mouse is irradiated before cell implantation; excising from the mouse after implantation a tumor emitting strong bioluminescence or other marker; culturing *in vitro* the bioluminescent portion of the tumor in the presence of a positive selecting agent for the cells; selecting a cell colony with high luciferase expression and culturing said cells. In another embodiment, the invention provides a tumor cell line that has been cured of replicon and demonstrates a decreased sensitivity to interferon alpha or another immunomodulatory compound as compared to its parental cell line that has not been passaged through the animal while harboring the replicon.

Various reporters and immunocompromisation methods can be utilized.

### BRIEF DESCRIPTION OF DRAWINGS

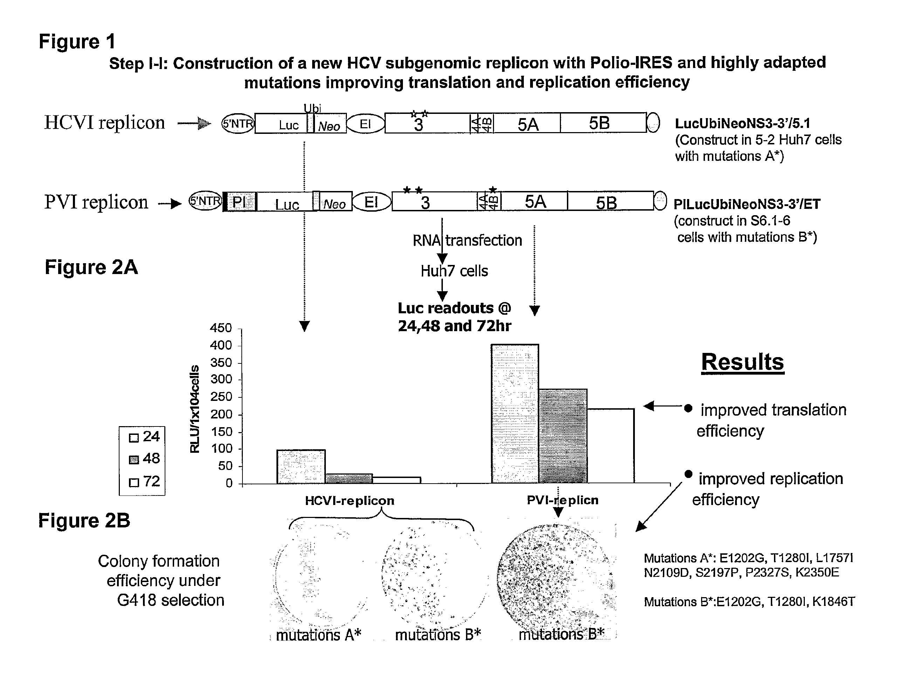

Fig. 1 shows a sub-genomic HCV replicon construct PILucUbiNeoNS3-3'/ET (a Polio Virus IRES replicon) that contains two Huh7 cell adaptive mutations E1202G and T1280I in the NS3 region and one adaptive mutation K1846T in NS4B region. The location of these adaptive mutations are indicated by stars in Fig. 1. The replicon shown is used in the animal models according to one embodiment of the present invention.
Fig. 2A is a graph showing the luciferase signal expressed in relative light units (RLUs) per 1x10⁴cells at 24, 48 and 72 hours post transfection with the PILucUbiNeoNS3-3'/ET replicon (labeled the PVI replicon in Fig. 1) in Huh7 cells and the LucUbiNeoNS3-3'/5.1 replicon (labeled the HCVI replicon in Fig. 1), which has the luciferase expression driven by the HCV 5'UTR/IRES, in Huh7 cells.
Fig. 2B shows colony formation of Huh7 cells containing the HCVI replicon (with adaptive mutations E1202G, T1280I, L1757I, N2109D, S2197P, P2327S and K2350E), Huh7 cells containing the HCVI replicon (with adaptive mutations E1202G, T1280I and K1846T) and cells containing the PVI replicon (with adaptive mutations E1202G, T1280I and K1846T indicated with stars in Fig. 1). Replicon-harboring cells were selected with G418.

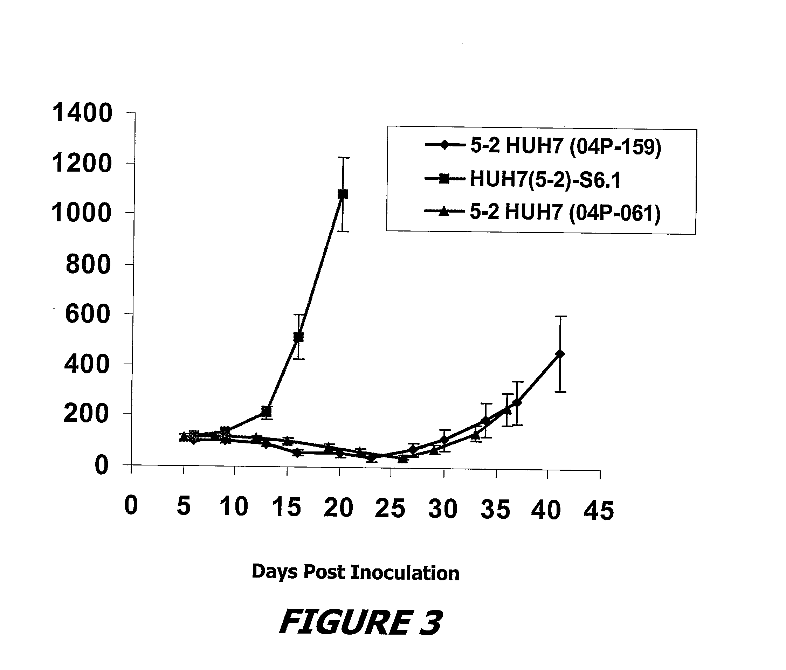

Fig. 3 is graphs showing results of two studies (the 061 and 159 studies) of the kinetics of tumor formation by 5-2 Huh7 cells and S6.1-6 cells. The 061 study determined the kinetics of tumor formation of 5-2 Huh7 cells and the 159 study determined the kinetics of tumor formation of S6.1 cells and 5-2 Huh7 cells.

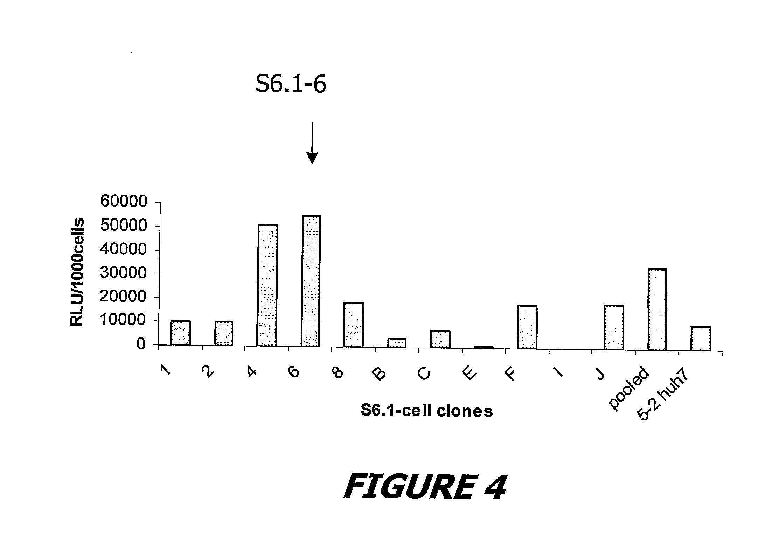

Fig. 4 shows results of a luciferase assay of S6.1 (fast-growing tumor) cell clones containing transfected RNA from Huh7 PILucUbiNeoNS3-3'/ET (i.e. the PVI replicon shown in Fig. 1).

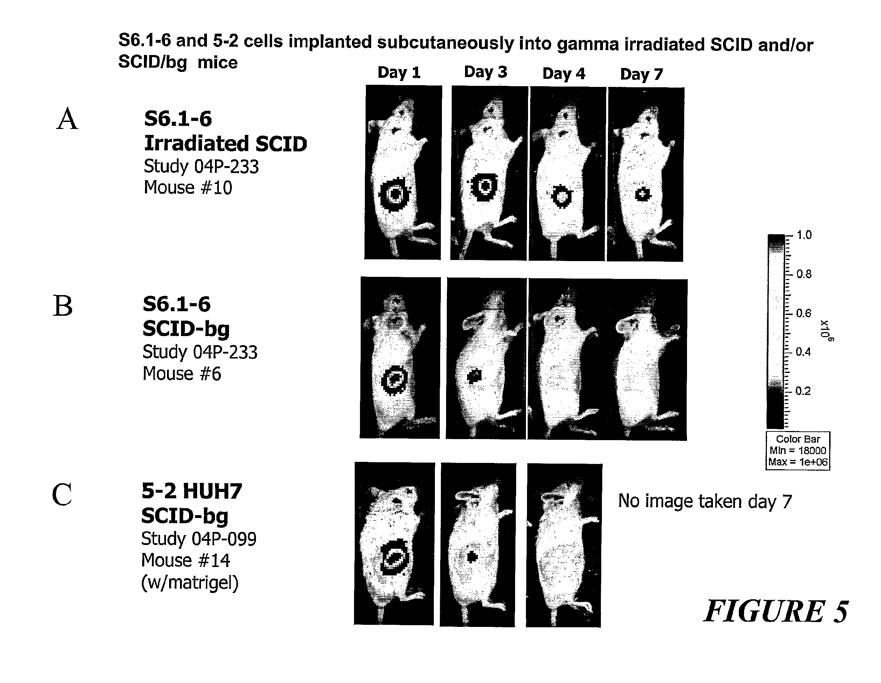

Figs. 5A-C are images of mice implanted with replicon-containing cells. Fig. 5A shows images of a gamma-irradiated SCID mouse subcutaneously implanted with S6.1-6 cells. Fig. 5B shows images of a SCID-bg mouse subcutaneously implanted with S6.1-6 cells. Fig. 5C shows images of a SCID-bg mouse subcutaneously implanted with 5-2 Huh7 cells in the presence of matrigel. Images were taken on days 1, 3, 4 and 7 post-inoculation and show bioluminescence.

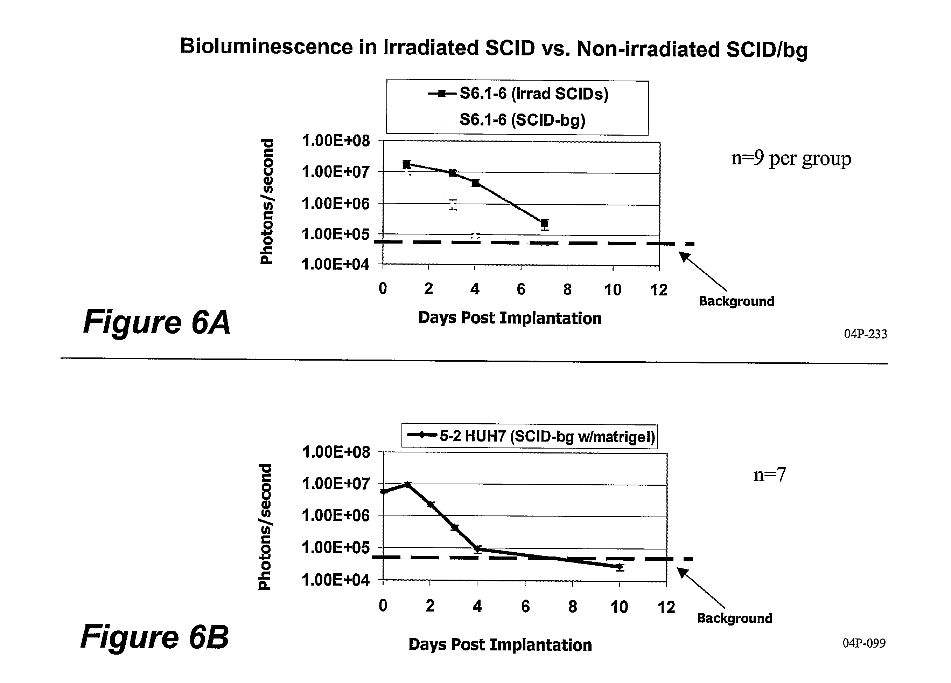

Figs. 6A and B are graphs showing mean bioluminescence as a function of time post-implantation for mice inoculated with the S6.1-6 or 5-2 Huh7 cells. Fig. 6A shows the mean bioluminescence of S6.1-6 cells subcutaneously implanted into gamma-irradiated SCID mice (a representative mouse is shown in Fig. 5A) compared with S6.1-6 cells subcutaneously implanted into SCID/bg mice (a representative mouse is shown in Fig. 5B). Fig. 6B shows the mean bioluminescence of 5-2 cells Huh 7 subcutaneously implanted in the presence of matrigel into SCID/bg mice (a representative mouse is shown in Fig. 5C).

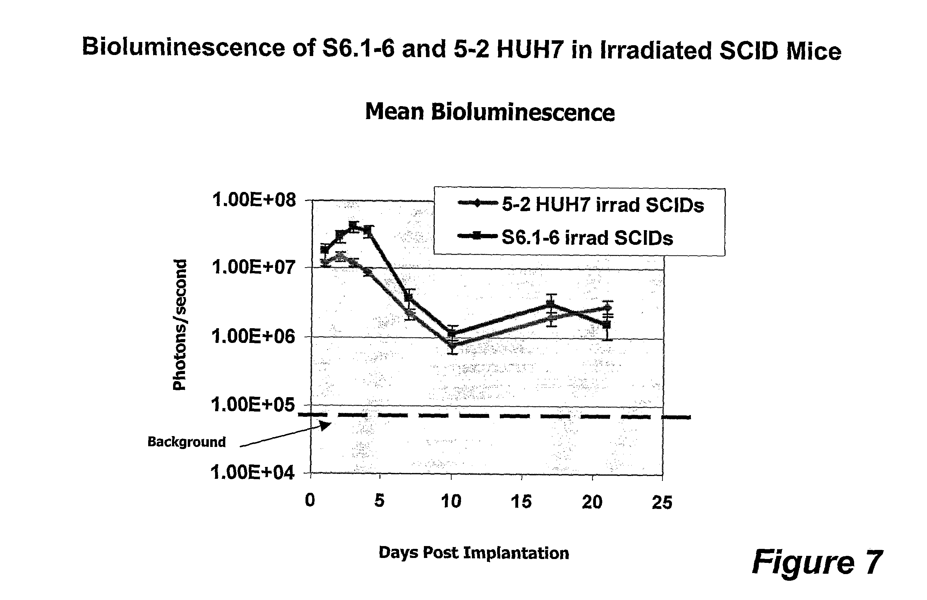

Fig. 7 is a graph comparing the mean bioluminescence on days 1, 2, 3, 4, 7, 10, 17 and 21 of gamma-irradiated SCID mice subcutaneously implanted with S6.1-6 cells with that of gamma-irradiated SCID mice subcutaneously implanted with 5-2 Huh7 cells.

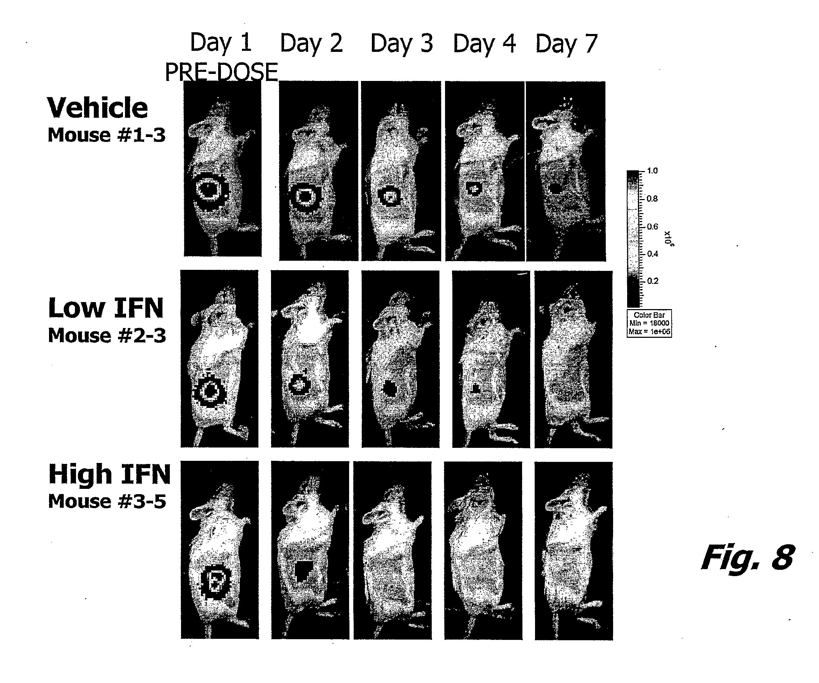

Fig. 8 are day 1, 2, 3, 4 and 7 images of representative mice from each of three experimental groups subcutaneously-implanted with S6.1-6 cells: a control group, a low dose IFN-alpha group and a high dose IFN-alpha group. The mice were gamma-irradiated and given the following dosages: control group: Vehicle (0.1 ml of 100 µµg/ml Human Serum Albumin in Hank's Balanced Salt Solution (HBSS) administered subcutaneously (SC), once per day (QD); low dose IFN-alpha group: 2,400 IU per day per 18 gram mouse IFN-alpha administered SC; high dose IFN-alpha group: 200,000 IU per day per 18 gram mouse IFN-alpha administered SC. Images were taken prior to dosing on each of the days.

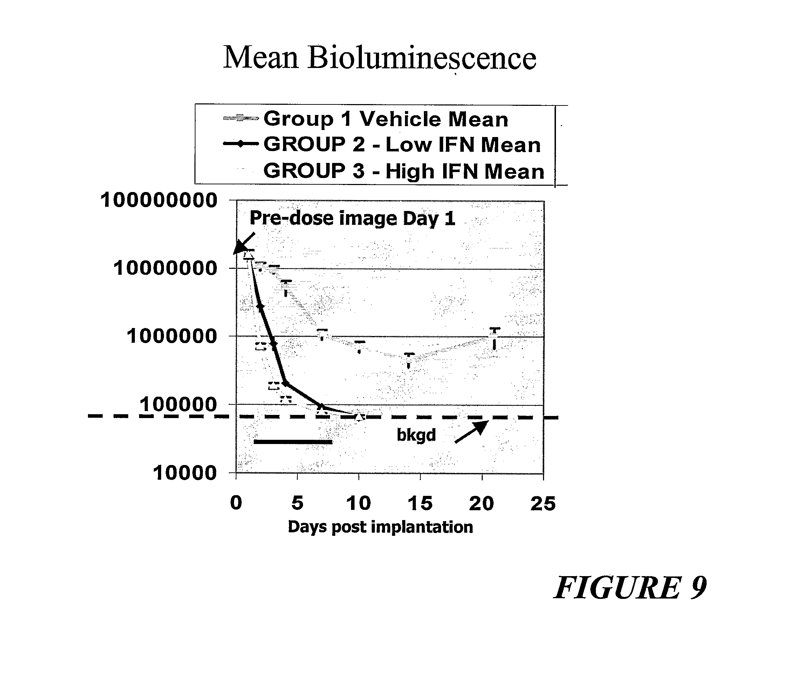

Fig. 9 is a graph showing the mean bioluminescence of each group described above in reference to Fig. 8. Bioluminescence was measured prior to daily dosing.

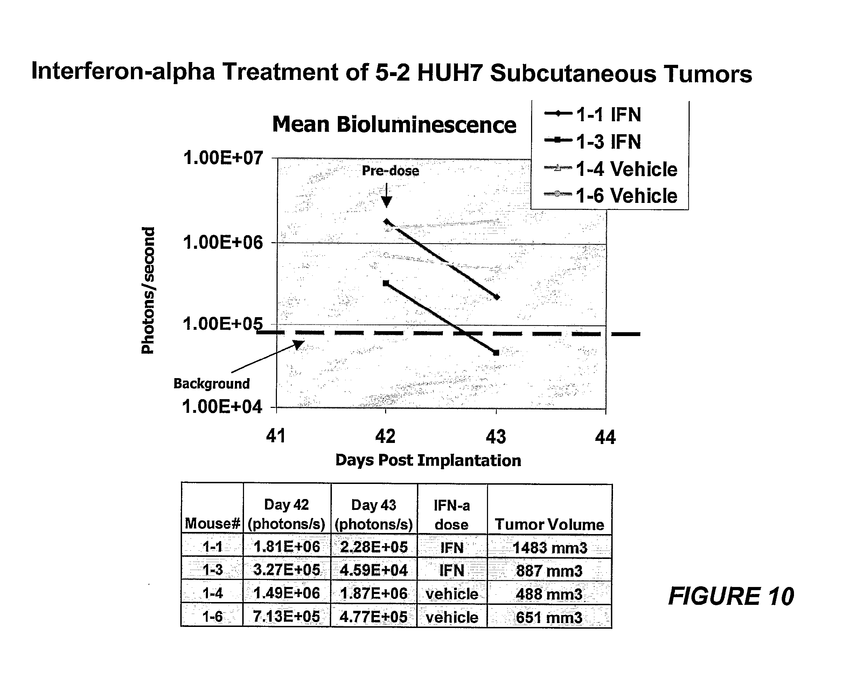

Fig. 10 is a graph showing bioluminescence signal of mice treated with IFN-alpha (IFN) compared with untreated mice (Vehicle) on day 42 after 5-2 Huh 7 cells were subcutaneously implanted in the mice and tumors were allowed to grow. On day 42, mice were given the following dosages: control group: Vehicle (0.1 ml of 100 µµg/ml Human Serum Albumin in HBSS) SC, QD; IFN-alpha group: 200,000 IU per day per 18 gram mouse IFN-alpha SC. Bioluminescence was measured prior to dosing and on day 43 after dosing.

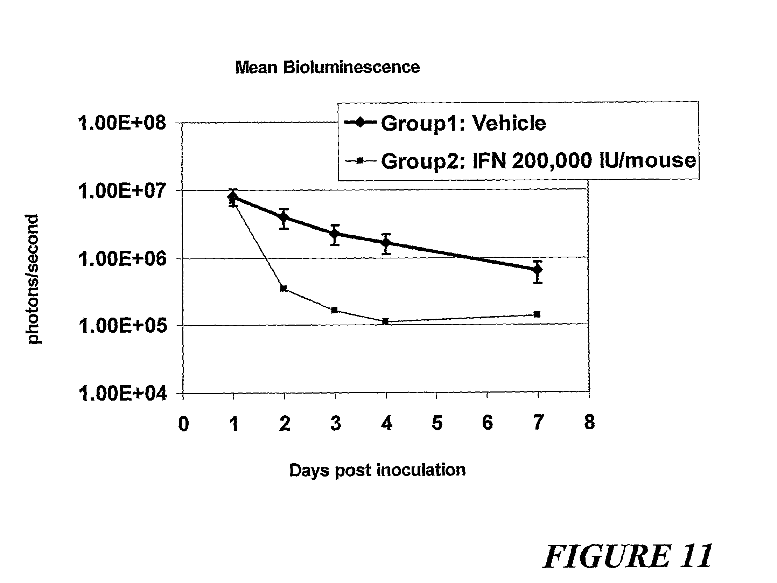

Fig. 11 is a graph showing mean bioluminescence of mice implanted S6-1-6 cells implanted into the liver measured over the course of 7 days. Mean bioluminescence of mice treated with IFN-alpha (IFN) is compared with that of untreated mice. The mice were treated with the following dosages: The vehicle or control group (group 1) was dosed with 0.1 ml of 100 µg/ml Human Serum Albumin in HBSS) SC per day, QD. The IFN-alpha group (group 2) was dosed with 200,000 IU IFN-alpha per day per 18 gram mouse SC. Bioluminescence was quantified on days 1, 2, 4 and 7 before dosing.

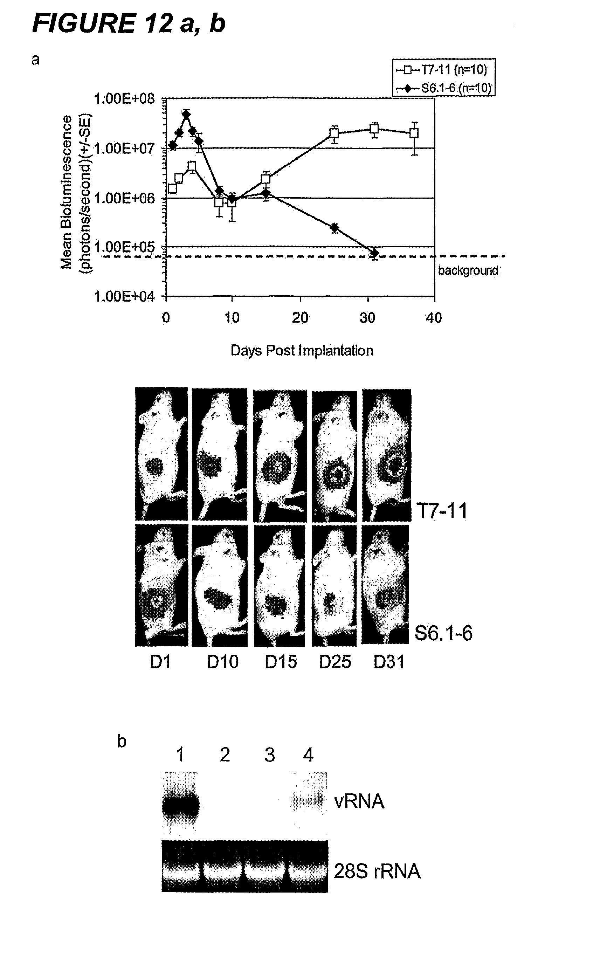

Figure 12 Correlation between mean bioluminescence and replication of subgenomic RNA in T7-11 SC model. (a) Mean bioluminescence of mouse adapted replicon-containing T7-11 cells and parental S6.1-6 cells in gamma irradiated SCID mice. T7-11 and S6.1-6 cells were implanted subcutaneously, mean bioluminescence was obtained at different time points and plotted in the graph. The images of representative mice from two groups at day 1, 10, 15, 25 and 31 are shown in the bottom panel. (b) Detection of HCV viral RNA in tumor tissues. Total RNA (10µg) was isolated from the tumors formed in gamma irradiated SCID mice 26 days post implantation with T7-11 cells subcutaneously (lane 4), and analyzed by Northern blot analysis. Blots were hybridized with radio-labeled RNA probes corresponding to the HCV NS5 region. 1x10⁸ genomes of *in vitro* transcribed HCV RNA(vRNA) served as a marker and control for the hybridization reaction (lane 1), and 28S rRNA served as a control for amount of RNA present in each sample analyzed. Lane 2 and 3 were total RNA extracted from naïve Huh7 and S6.1 cells as negative control.

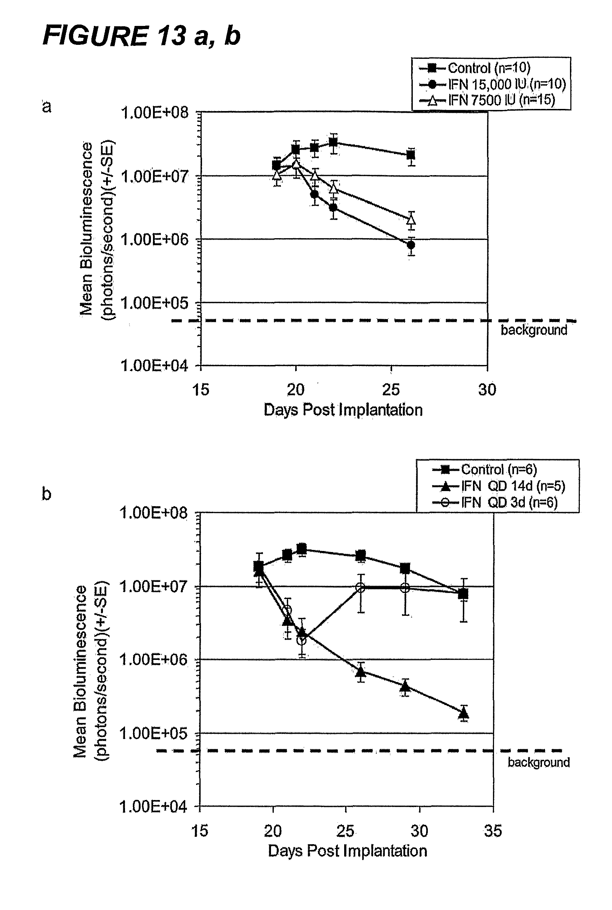

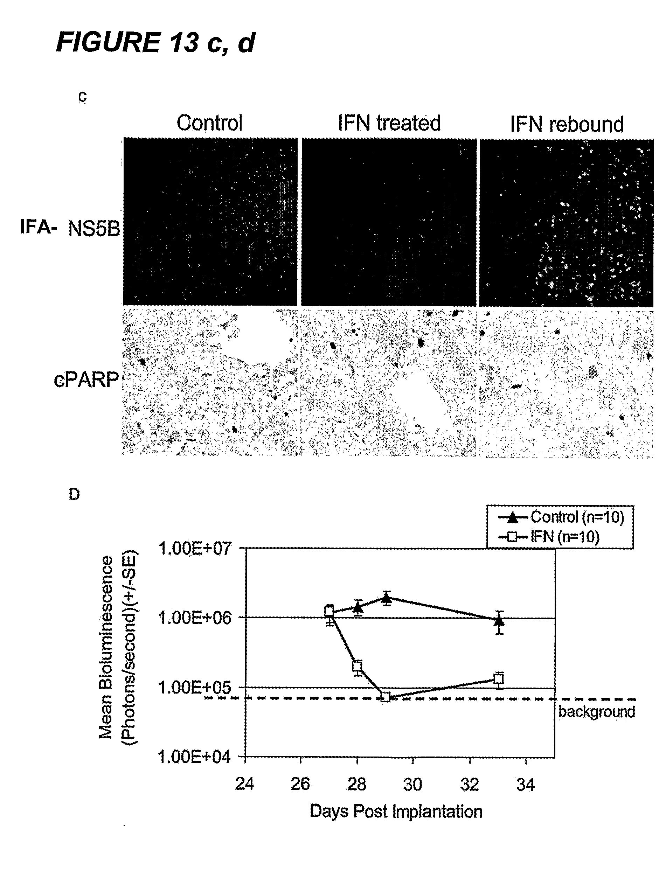

Figure 13 Antiviral activity of IFN-α 2B (IFN) in T7-11 model. (a) Dose-dependent IFN anti-HCV effect in SC mouse model. IFN treatment was given at day 19 post implantation with T7-11 cells subcutaneously in gamma irradiated SCID mice. The following dosages were administered once daily for seven days by subcutaneous route: control, 0.1ml of 100µg/ml human albumin in HBSS; 7,500IU group, 7,500IU of IFN per day per 18gram mouse; 15,000IU group, 15,000IU of IFN per day per 18gram mouse. (b) IFN treatment was given same as described in (a) with 15,000IU/mouse/day in two groups: IFN QD 14 days, once daily dosing for two weeks; IFN QD 3 days, once daily dosing for three days. Individual mice were imaged at day 19 before dosing, then on days 21, 22, 26, 29 and 33. (c) Immunohistochemical analysis of HCV replication and cell death in the tumor tissues from untreated, IFN treated (IFN QD 14 days) and IFN rebound (IFN QD 3 days) groups 33 days post implantation as described in (b). Protein expression of NS5B was detected with a monoclonal antibody bound to fluorescence isothiocyanate-conjugated antibody against NS5B. Cell death was detected with cPARP antibody as described in Methods (d) IFN anti-HCV efficacy study in liver mouse model. T7-11 cells were implanted directly into one lobe of the liver of γ-irradiated SCID mice. IFN treatments were given SC once daily for 2 days (gray line) starting on day 27 post implantation. 15,000IU/mouse/day was given to the treated group, and human albumin for control group. Data are representative of two to four experiments for each panel. Error bars indicate standard error (SE).

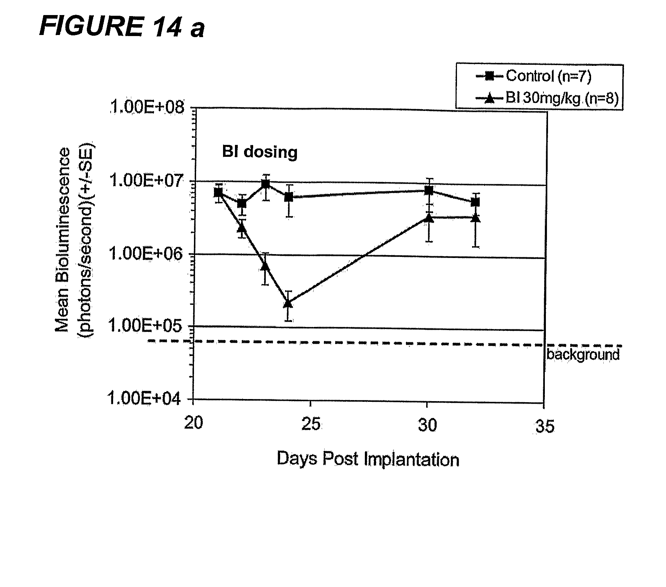

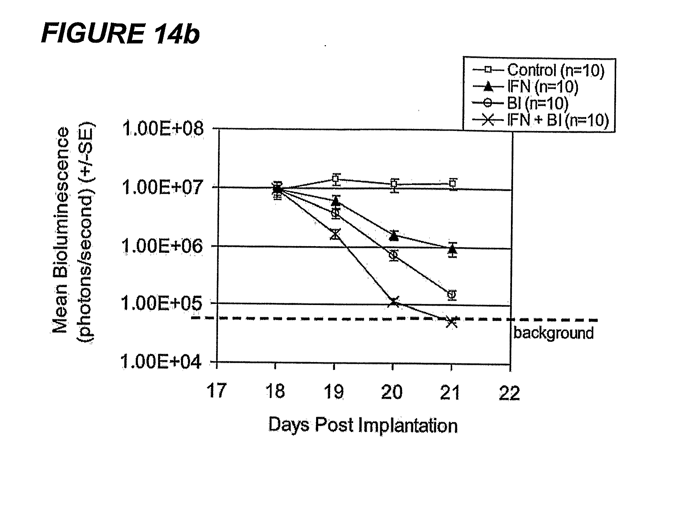

Figure 14 Antiviral effect of protease inhibitor BILN 2061(BI) alone in the T7-11 SC model. (a) T7-11 cells were implanted subcutaneously into γ-irradiated SCID mice. BILN 2061 (BI) treatments were given at 30mg/kg once daily for three days (gray line) starting on day 21 post implantation. BI treatment was withdrawn on day 24. Individual mice were imaged at day 21 before dosing, then on days 22, 23, 24, 30 and 32. (b) T7-11 cells were implanted subcutaneously into γ-irradiated SCID mice. BI at 30mg/kg and/or IFN at 15,000IU per mouse were given starting on day 18 post cell implantation. Bioluminescence was measured every day for four days.

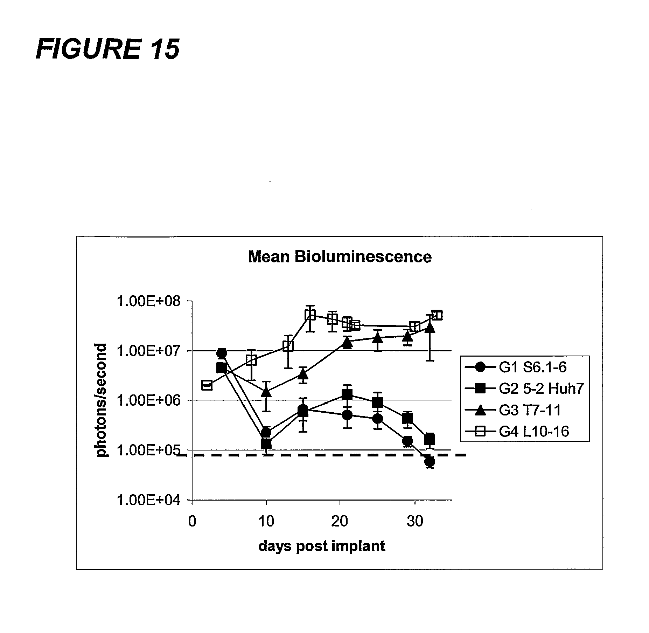

Figure 15 shows a graphical representation of replicon expression following subcutaneous administration of various replicon harboring cell lines.

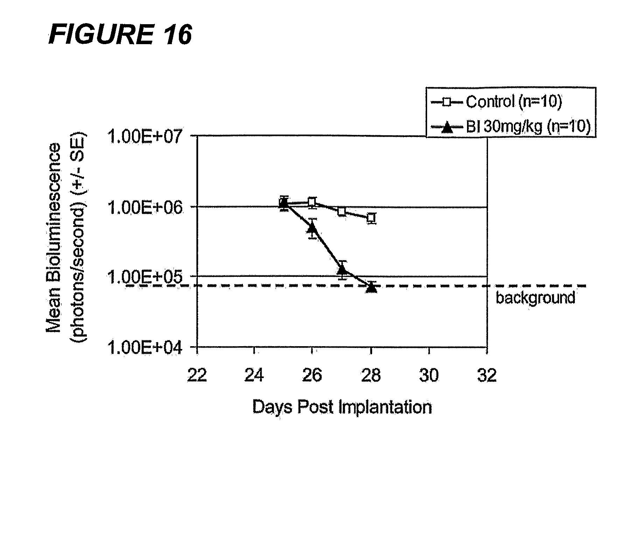

Figure 16 shows the antiviral effect of BILN 2061 on HCV replication. BILN 2061 was administered subcutaneously at 30mg/kg once daily starting day 25-post tumor cell implantation. A 10-fold decline in bioluminescence within three days was observed in the treated group compared to the untreated control group (P<0.0001) **(****Fig. 16****).**

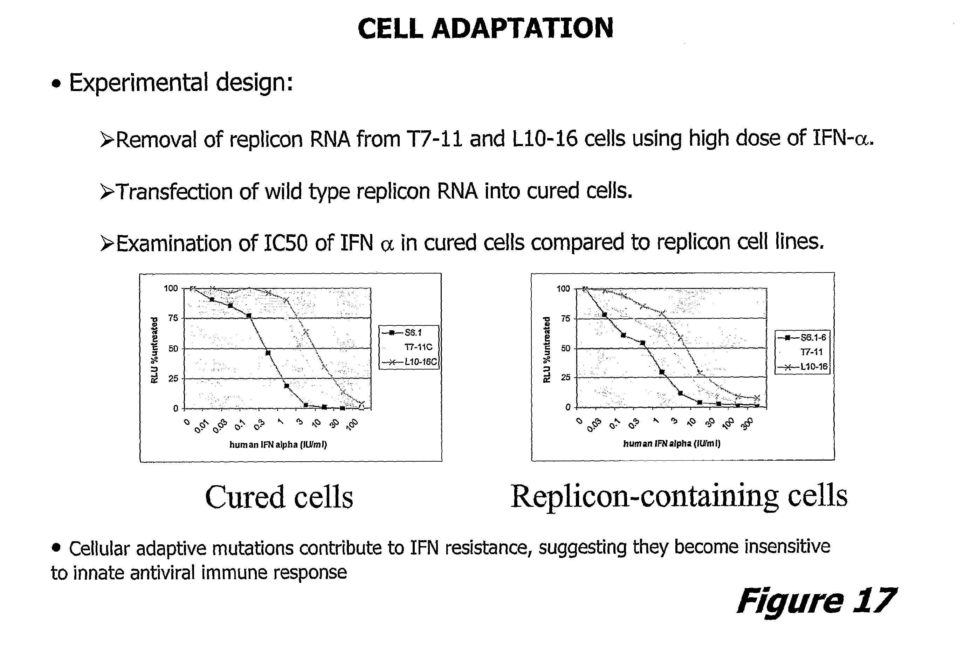

Figure 17 demonstrates that cellular adaptations are responsible for increased resistance to α-interferon in replicon cell lines.

### DETAILED DESCRIPTION

The present invention provides an animal that can be used as an animal model for HCV replication and/or the production of viral or virus-like particles. The model uses animals having cells containing an HCV replicon comprising a reporter gene. The cells are transfected with an HCV replicon that supports the expression of viral and non-viral proteins including a reporter gene such as, *e.g*., a luciferase gene, linked to the replication and or expression of the replicon. After introducing cells containing the HCV replicon into the animal, the reporter gene allows HCV replicon replication expression or viral particle production to be measured *in vivo* or *in vitro.* According to various embodiments, human-derived cells that are adapted for growth and/or replicon-stability in the host may be used. The animal model may be used as an assay to evaluate potential activity of HCV anti-viral compounds *in vivo* or *in vitro.*

Some advantages of the model are the following: it does not require transgenic animals; it may be used with readily-available animals (such as SCID mice); and it permits human-derived cells to be used. Additionally, the model permits the use of a non-infectious HCV replicon, unlike previous models, which describe the use of infectious human innocula. The animal thus provides a safer and more convenient model for screening and detecting potential anti-HCV therapeutic agents.

The cells containing the replicon can be introduced or implanted into various target sites in the animal. The cells can be implanted in or delivered to the liver and/or the subcutaneous space of the animal. In some embodiments, the introduced cells form tumors. The cells can be transfected with the replicon in vitro or can be transfected in vivo after being introduced into the animal.

The present model of HCV replication can be used to monitor *in vivo* and/or *in vitro* the replication or expression of an HCV genomic or subgenomic replicon by detecting or monitoring the replication or expression of a reporter gene contained within the replicon.

The model also provides means for monitoring one or more essential steps in the life cycle of HCV by detecting or monitoring reporter gene activity present in HCV virus or virus-like particles produced from cells containing the replicon. The virus life cycle includes the steps required for a virus to enter into a cell, produce virus particles and exit the cell in order to infect other cells. These steps include binding to the cell surface, interacting with receptors on the surface which facilitate cell entry, uncoating the virus particle to release the viral genome, replication and expression of the viral genome, particle maturation including packaging the viral genome into a particle, egress through the cell and finally exiting the cell and the release of viral progeny. (See Fields Virology (1996) Chapter 10: Virus-Host Interactions D. Knipe).

The model may be used as an *in vivo* assay to evaluate potential HCV antiviral compounds. In one embodiment, the animal is administered a compound known to reduce or prevent HCV replication and or particle formation and then a second candidate compound is administered to the animal to determine whether the second candidate compound has an additive or synergistic effect with the known compound in reducing or preventing HCV infection. The model may also be used to measure replication and expression of the HCV replicon *in vitro* in cells containing an HCV replicon as described herein that were previously introduced and subsequently removed from the animal.

In another embodiment, the invention provides pharmaceutical compositions and compounds and combinations of compounds for the treatment or prevention of HCV in mammals. The pharmaceutical compositions can include compounds that are administered simultaneously or sequentially.

The pharmaceutical compositions may be provided as components in a kit.

The pharmaceutical compositions can include one or more carriers and an excipient

In another embodiment, the invention provides methods of treating or preventing HCV in mammals by administering the compounds or combinations of the invention. Pharmaceutical compositions of the invention include one or more pharmaceutical carrier(s) and/or excipient(s).

To control tonicity, it is preferred to include a physiological salt, such as a sodium salt. Sodium chloride (NaCl) is preferred, which may be present at between 1 and 20 mg/ml.

Compositions will generally have an osmolality of between 200 mOsm/kg and 400 mOsm/kg, preferably between 240-360 mOsm/kg, and will more preferably fall within the range of 290-300 mOsm/kg.

Compositions of the invention may include one or more buffers. Typical buffers include: a phosphate buffer; a Tris buffer; a borate buffer; a succinate buffer; a histidine buffer; or a citrate buffer. Buffers will typically be included in the 5-20mM range.

The pH of a composition of the invention will generally be between 5.0 and 7.5, and more typically between 5.0 and 6.0 for optimum stability, or between 6.0 and 7.0. The process of the invention may therefore include a step of adjusting the pH of the bulk vaccine prior to packaging.

Compositions of the invention are preferably sterile.

Compositions of the invention are preferably non-pyrogenic *e.g*. containing <1 EU (endotoxin unit, a standard measure) per dose, and preferably <0.1 EU per dose. Compositions of the invention are preferably gluten free.

Compositions of the invention may be prepared in various forms. For example, the compositions may be prepared as injectables, either as liquid solutions or suspensions. Solid forms suitable for solution in, or suspension in, liquid vehicles prior to injection can also be prepared (*e.g*. a lyophilised composition, like Synagis™ and Herceptin™, for reconsitution with sterile water containing a preservative). The composition may be prepared for topical administration *e.g*. as an ointment, cream or powder. The composition may be prepared for oral administration *e.g*. as a tablet or capsule, as a spray, or as a syrup (optionally flavoured). The composition may be prepared for pulmonary administration *e.g*. as an inhaler, using a fine powder or a spray. The composition may be prepared as a suppository or pessary. The composition may be prepared for nasal, aural or ocular administration *e.g.* as drops. The composition may be in kit form, designed such that a combined composition is reconstituted just prior to administration to a patient. For example, a lyophilised antibody can be provided in kit form with sterile water or a sterile buffer. Compositions of the invention will generally be in aqueous form.

Compositions of the invention may be administered to patients in 0.5ml doses. References to 0.5ml doses will be understood to include normal variance *e.g*. 0.5ml+0.05ml.

Pharmaceutical compositions include an effective amount of one or more antiviral compounds in an amount sufficient to treat, ameliorate, or prevent a desired disease or condition, or to exhibit a detectable therapeutic effect. Therapeutic effects also include reduction in physical symptoms. The precise effective amount for any particular patient will depend upon their size and health, the nature and extent of the condition, and the therapeutics or combination of therapeutics selected for administration. The effective amount for a given situation is determined by routine experimentation and is within the judgment of a clinician. For purposes of the present invention, an effective dose will generally be from about 0.01mg/kg to about 50mg/kg, or about 0.05 mg/kg to about 10 mg/kg. Known antibody-based pharmaceuticals provide guidance in this respect *e.g*. Herceptin™ is administered by intravenous infusion of a 21 mg/ml solution, with an initial loading dose of 4mg/kg body weight and a weekly maintenance dose of 2mg/kg body weight; Rituxan™ is administered weekly at 375mg/m²; *etc*.

The invention is particularly suitable for therapy in human patients.

In some embodiments, rodents are used and, in one preferred embodiment, mice are used. The animal may be immuno-compromised by genetic mutation (*e.g.* SCID mice), by irradiation, by administration of biological or chemical immuno-suppressants (*e.g*. a gluco-corticoid) or a combination thereof

Other small animals, as described herein, can also be used.

The cells introduced into the animal may be any cells capable of harboring an HCV replicon, but are preferably immortalized human-derived cells, and in one embodiment are human hepatoma Huh7 cells. According to various embodiments, the replicon transfected into the cell line may be genomic or sub-genomic. The replicon may be transcribed from an antigenomic (or anti-subgenomic) template. In one embodiment, the replicon is a sub-genomic replicon expressing non-structural HCV proteins required for replication. Also, the replicon includes a reporter gene that allows *in vivo* or *in vitro* measurement of the reporter gene activity. In one embodiment luciferase is the reporter.

The reporter gene is contained within the HCV replicon and is replicated when the replicon is replicated. The reporter gene may be co-expressed with the genes encoding the HCV proteins, either as a mono-cistronic, bicistronic or tricistronic RNA. In the bi-cistronic configuration, one or more genes within the replicon is under the translational control of a separate genetic element that does not regulate expression of the other genes also present on the replicon. An additional gene or genes may be expressed (1) with the HCV protein(s), (2) with the reporter gene or (3) under the control of yet another separate translational control element contained in the replicon (*i.e*., the "tricistronic" configuration). Furthermore, the HCV proteins can be expressed together as an HCV polyprotein or may be under the expression of separate genetic elements as described above for the reporter gene and any additional gene(s). Alternatively, replication can be detected without protein expression of the reporter gene by, for example, by assaying for the relative presence or absence of a nucleic acid that encodes reporter specific nucleic acid sequences.

Genetic elements that regulate translation of genes are well known to the skilled artisan and include, e.g., Internal Ribosome Entry Site (IRES) from HCV, Poliovirus and Encephalo-myocarditis virus.

In another aspect, the invention provides a method of identifying a compound having anti-HCV activity, i.e. a compound that reduces or inhibits the level of HCV replication and or expression of HCV proteins. A compound can directly regulate replication by interfering with replication of the HCV replicon in the host or can indirectly regulate replication by interfering with expression of HCV proteins that are necessary for HCV replication in the host. Alternatively, as discussed below, the animal provides a method of identifying compounds that have no effect on replication or expression per se, but instead reduce or inhibit virus or virus-like particle formation.

The *in vivo* replication and or expression of the HCV replicon can be directly detected *in vivo* by detecting reporter gene activity in the animal. Alternatively, reporter gene activity resulting from *in vivo* replication or expression in the animal can be detected in the blood or tissue of the animal after the blood or tissue are removed from the animal.

In one embodiment, an animal, having a cell or cells containing an HCV replicon further comprising a reporter gene, is contacted with a test compound *in vivo.* The reporter gene is only replicated if the replicon is replicated. As discussed herein, depending on the embodiment, expression of the reporter gene protein does not have to, but may optionally be linked to expression of the HCV polyprotein. Activity of the reporter gene contained within the replicon in the animal is compared to activity of the reporter gene contained within the replicon in an animal containing the cell or cells that is not contacted with the test compound. A compound that reduces replication and or expression of the replicons is a compound that may have use as a medicament for the regulation of HCV replication and or expression in a human.

In another embodiment, an animal, having a cell or cells containing an HCV replicon further comprising a reporter gene is contacted with a test compound *in vivo.* Activity of the reporter gene contained within the virus or virus-like particle in the animal is compared to activity of the reporter gene contained within the virus or virus-like particle in an animal containing the cell or cells that is not contacted with the test compound. A compound that reduces or inhibits activity of the reporter gene contained within the virus or virus-like particle but has no effect on replication or expression of the replicon is a compound that may have use as a medicament for the regulation of HCV Virus or Virus-like particle formation.

In another embodiment, the cell or cell containing the HCV replicon is introduced into an animal. The cells form tumors and the tumor cells contain the replicon. After replication *in vivo,* cells containing the replicon are surgically removed from the animal, grown in tissue culture and contacted with the test compound *in vitro.* HCV replication and or expression are compared *in vitro* to a cell or cells containing the HCV replicon that are cultured in the same manner but not contacted with the test compound. A compound that reduces or inhibits replication and or expression may be useful in the manufacture of a medicament useful for treating HCV infection.

When the reporter gene contained in the HCV replicon is provided in the monocistronic configuration, it is expressed as part of the polyprotein produced by the genomic or subgenomic replicon. The reporter gene can be cleaved from the polyprotein by providing cleavage recognition sites at both boundaries of the reporter gene product (or at one boundary if the reporter gene product is at the amino or carboxyl terminus of the polyprotein). The cleavage sites are recognized by a protease that specifically cleaves at the sites (*e.g*., the HCV protease). Alternatively, the reporter gene can be constructed without cleavage site(s) and can thus be maintained as a fusion protein fused to one of the HCV proteins. In a genomic replicon, when the reporter gene is part of a fusion protein it is fused to a structural protein (*i.e*., core or envelope). In a subgenomic replicon, when the reporter gene is part of a fusion protein, it is fused to a non-structural protein. In one preferred embodiment, when the reporter gene is contained within a subgenomic replicon having a fusion protein, the reporter is fused to NS5A. It is not necessary that the reporter gene always must express a protein. In some embodiments, the reporter can be detected by detecting the relative presence or absence of a nucleic acid sequence specific for the reporter.

The animal provides an animal model of HCV replication that may be used for evaluation and identification of inhibitors of HCV replication, for pharmacokinetic (PK) and pharmacodynamic (PD), toxicity studies and rapid screening of anti-viral HCV candidate compounds *in vivo.*

### Replicon

The term "replicon" refers to a viral nucleic acid that is capable of directing the generation of copies of itself when present in a cell. Cell-based HCV replication systems that use a genomic or sub-genomic replicon system are well known. Cells containing the replicon as described herein are introduced into mice or other animals according to the present invention. The cells can additionally contain additional viral replicons and or plasmids containing or expressing additional nucleic acids, genes or proteins. One or more other additional genes and/or regulatory regions can also optionally be present and expressed in a cell transfected with the HCV replicon). Additional genes or proteins can be provided on the HCV replicon or can be stably or transiently transfected into the host cell genome using various techniques well known in the art. An additional gene or protein can be used *e.g*., as a marker for cell survival. Furthermore, an additional gene or protein may provide an accessory function that enhances or enables HCV replication, expression and/or virus or virus like particle production.

An endogenous host gene may be over-expressed, either by up-regulating expression of a genomic gene or by providing a further copy of the gene in another delivery vector or integrated into the chromosome. Conversely, down-regulation of inhibitory proteins can be used. Methods for down-regulation of expression include use of an antisense nucleic acid, a ribozyme, a locked nucleic acid, micro RNA or a siRNA One protein which may have use as an accessory factor for providing an accessory function is the host cell is caveolin-3, and so delivery of an additional heterologous caveolin-3 or up-regulation of endogenous caveolin-3 can be used with the invention. Caveolin-3 has been reported to be a chaperonin.

It has now been found that caveolin-3 expression is seen not only in muscle. Surprisingly, it has been detected in the Huh-7 cell line that carries a HCV replicon, and has moreover been found to co-localise in the cell with particulate HCV protein structures. Expression seems not to be induced by HCV infection, but to exist at a low endogenous level. Caveolin-3 appears to play a role in HCV maturation.

The GeneCard entry for caveolin-3 can be found using code CAV3. The GeneCard reveals that the protein has also been referred to as LGMD1C, M-caveolin and VIP-21. Ten SNPs have been described so far. The reference human CAV3 sequence is a 151-mer having GI number GI:4502589 and accession number NP_001225 (SEQ ID NO: 1):

As used herein, the term "additional gene" is meant to encompass a gene or other nucleic acid that expresses a protein or provides a regulatory function in the cell. The additional gene can be hetorologous to HCV and the host cell or may be from HCV or the host cell genome. Thus, as used herein, the term can mean a host cell gene that is already present in the host cell genome, but is provided as an additional gene either as an episomal element or integrated into a site on the host cell genome distinct from (and in addition to) its normal location.

As used herein, the singular form can include the plural and the plural can include the singular, unless specifically indicated otherwise. Therefore, for example, reference to "a cell" can include a plurality of cells and reference to "cells" can include a cell, unless specifically indicated otherwise. Similarly, transfection of a cell with a replicon is meant to include the situation where a plurality of cells are transfected with a plurality of replicons. Similarly, the phrase "introducing a cells containing the replicon into the animal" is meant to include the situation where a plurality of cells containing the replicon are produced into the animal.

It should be understood that the terms "replicon" and "genetic element" encompass the sense strand of the viral RNA or any complementary sequence (*e.g*. a cDNA reverse transcript or complementary RNA) that can be converted into the sense strand (the viral genomic RNA) and can be translated into the viral proteins required for replication. Those of skill in the art will understand that alternate constructs may be used.

Sub-genomic HCV replicons lack one or more regions of the HCV genome that encode at least one HCV protein or functional fragment of the protein. The subgenomic replicons are non-infectious.

Sub-genomic HCV replicons expressing HCV one or more non-structural proteins (e.g., protease polymerase), but not all HCV structural proteins, are known in the art. For example, in one system, human hepatoma cell line 5-2 Huh7 bears a replicon expressing the non-structural proteins NS3, NS4A, NS4B, NS5A and NS5B [Nicole Krieger, Volker Lohmann, and Ralf Bartenschlager. Enhancement of hepatitis C virus RNA replication by cell culture-adaptive mutations. 2001, Journal of Virology, 75(10): 4614-4624]. The sub-genome is replicated and expresses at least some HCV non-structural proteins in the cell line.

In some embodiments, the replicon is a sub-genomic replicon that replicates and expresses at least one HCV non-structural protein, a selectable marker and a reporter. The selectable marker allows for the selection of replicon-harboring cells. In some embodiments neomycin phosphotransferase gene (Neo) for selection with G418 is used. Examples of other markers based on drug resistance include *aad, ble, dhfr, hpt, nptII, aphII, gat,* and *pac.* Other markers may allow selection using antibiotics such Puromycin, Zeocin or Hygromycin, but one of skill in the art will understand that any marker gene known in the art that allows for selection of the replicon-harboring cells may be used.

In one embodiment, the reporter is luciferase. Reporters and reporter genes are discussed in further detail below. The reporter gene or genes can be used to monitor HCV replication and/or cell viability *in vitro* or *in vivo.*

The HCV replicon used in the animal models of the present invention may be infectious, produce virus or virus-like particles, or may be non-infectious.

The replicon may have one or more cell-adaptive mutations. These mutations can enhance or allow HCV replication in cells that are either non-permissive or otherwise not efficient for HCV production. In one embodiment, wherein Huh7 cells are used, the replicon can have the following cell-adaptive mutations: E1202G, T1280I in the NS3 region and K1846T in NS4B region [Nicole Krieger, Volker Lohmann, and Ralf Bartenschlager, Enhancement of hepatitis C virus RNA replication by cell culture-adaptive mutations. 2001, Journal of Virology, 75(10): 4614-4624; Volker Lohmann, Sandra Hoffmann, Ulrike Herian, Francois Penin, and Ralf Bartenschlager, Viral and cellular determinants of hepatitis C virus RNA replication in cell culture. 2003, Journal of Virology, 77(5):3007-3019]. Such cell-adaptive mutations are not absolutely required, however, as some cells can support HCV replication without having been adapted in cell culture (e.g., HCV-N or JFH-1). The models and animals of the invention can support growth of HCV replicons with or without these mutations.

The replicon preferably comprises an internal ribosome entry site (IRES). Suitable IRES are known from poliovirus, from and encephalo-myocarditis virus (EMCV), and from HCV itself Replicons that include the poliovirus IRES ('PVI') may be referred to as PVI replicons. Fig. 1 shows replicon PILucUbiNeoNS3-3'/ET. The PVI is upstream of and drives expression of the luciferase reporter gene (Luc). The EMCV IRES (EI) directs translation of the NS3 to NS5B region that is flanked at the 3' end by the 3' NTR. The replicon has a Neo selectable marker. Notably, Luc expression is driven by the PI rather than the HCV 5'NTR. The stars indicate adaptive mutations E1202G and T1280I in the NS3 region and K1846T in NS4B region.

The sub-genomic replicon with PVI and/or highly adapted mutations may provide improved translation and replication efficiencies over replicons without the mutations and/or PVI. For example, the construct used in 5-2 Huh7 cells is LucUbiNeoNS3-3'/5.1. Fig. 2A shows the luciferase readouts expressed as relative light units (RLU) per 10,000 cells of Huh7 cells transfected with the PILucUbiNeoNS3-3'/ET replicon as compared to LucUbiNeoNS3-3'/5.1 at 24, 48 and 72 hours after transfection. The PILucUbiNeoNS3-3'/ET replicon is labeled PVI replicon because Luc expression is driven by the poliovirus IRES. The LucUbiNeoNS3-3'/5.1 replicon is labeled HCVI replicon because Luc is driven by the 5'NTR region (an IRES) of the HCV replicon. The luciferase readouts indicate that the PVI replicon has improved translation efficiency over the HCVI replicon.

Fig. 2B shows colony formation efficiency under G418 selection. Huh7 cells harboring a HCVI replicon (with adaptive mutations E1202G, T1280I, L1757I, N2109D, S2197P, P2327S and K2350E), Huh7 cells harboring a HCVI replicon (with the adapted mutations E1202G, T1280I and K1846T) and Huh7 cells harboring a PVI replicon (with adapted mutations E1202G, T1280I and K1846T) were grown. Replicon-harboring cells were selected with G418. As can be seen in Fig. 2B, more of the PVI replicon-harboring cells have replicated than have the HCVI replicon-harboring cells. In addition, HCVI cells containing the mutations have improved replication over HCVI cells without the adapted mutations.

The animal model can comprise the replicon shown in Fig. 1 containing Huh7 cell-adaptive mutations and a poliovirus IRES, as it results in improved translation and replication efficiencies. However, the invention may be practiced with any HCV genomic or sub-genomic replicon or other genetic element expressing the HCV non-structural proteins such as protease and/or polymerase. This includes replicons or other genetic elements that do not contain adapted mutations as well as those that do.

HCV genomic and sub-genomic replicons may be derived from various viral strains and genotypes. The HCV genome can be of any type (*e.g*. 1,2,3,4,5,6) or subtype (*e.g.* 1a, 1b, 1c, 1d, 1e, 1f, 1g, 1h, 1i, 1j, 1k, 1l, 1m, 2a, 2b, 2c, 2d, 2e, 2f, 2g, 2h, 2i, 2k, 21, 2m, 3a, 3b, 3c, 3d, 3e, 3f, 3g, 3h, 3i, 3k, 4a, 4c, 4d, 4e, 4f, 4g, 4h, 4k, 4l, 4m, 4n, 4o, 4p, 4q, 4r, 4s, 4t, 5a, 6a, 6b, 6d, 6f, 6g, 6h, 6i, 6j, 6k, 6l, 6m, 6n). This nomenclature is the current standard, as set out by the NIAID *Hepatitis C Virus (HCV) Sequence Database* [*http:*//*hcv.lanl.gov*/*content*/*hcv-db*/*classification*/*genotable.html*] in which previous genotypes 7-9 have been reclassified as subtypes of type 6 [Simmonds et al. (1996) J Gen Virol 77:3013-24]. Thus the new classification includes previous classifications I, II, III, IV, V, VI, 4α, 4β, 7a, 7b, 7c/NGII/VII, 7d, NGI, 8a, 8b, 9a, 9b, 9c, 10a/ID3 and 11a. One suitable HCV strain for use with the invention is the genotype 2a strain JFH1 [Kato et al. (2003) J Med Virol 64:334-9.].

RNA from the HCV genome can be used to make a complementary DNA copy. A DNA copy can be made by reverse transcription (RT). Alternatively, a DNA copy, or fragment thereof, can be made entirely or in part by synthetic means. Once a DNA copy of the RNA genome is available, it can be converted to double stranded DNA and can then be cloned into a vector (*e.g*. a plasmid), and the plasmid can be used to make DNA templates from which viral RNA can be transcribed. As an alternative, the DNA may be used to generate viral RNA without using a vector, in which case a suitable promoter for a DNA-dependent RNA polymerase will be introduced upstream of the start of the viral genome. Suitable promoters include the T7, T3 and SP6 promoters, which are used in *in vitro* transcription ('IVT') reactions. DNA copies of the virus can optionally be amplified *e.g*. using the polymerase chain reaction (PCR).

As an alternative, viral transcripts can be made from an RNA template using an RNA-dependent RNA polymerase, such as Qβ replicase. The transcription mediated amplification ('TMA') reaction may be used to amplify RNA.

Plasmids containing HCV sequences may be constructed by any method well-known in the art. See, e.g., Sambrook, Fritsch, and Maniatis, Molecular cloning a laboratory manual (2nd Edition) 1989 Cold Spring Harbor Laboratory Press; Joseph Sambrook, David W. Russell, Joe Sambrook, Molecular Cloning: a Laboratory Manual, ed. Cold Spring Harbor Laboratory Press, 2000 or PCR Applications, 1999, ed. M. Innis, D. Gelfand and J. Sninsky, Academic Press. The PILucUbiNeoNS3-3'/ET replicon shown in Fig. 1 may be constructed by replacing the 2.6 kb XbalI-NotI fragment from pFK I 1389 LucUbiNeoNS3-3'/5.1 (Krieger Nicole, Lohmann Volker, and Bartenschlager Ralf. Enhancement of hepatitis C virus RNA replication by cell culture-adaptive mutations. 2001, Journal of Virology, 75(10): 4614-4624) with the Xbal-NotI fragment from pFK I 341 PI Luc NS3-3'/ET (available from ReBLikon).

### Reporter Genes

The animal of the present invention allows for the following of the anti-HCV activity of compounds in real-time and on a repetitive basis so that the hosts do not have to be sacrificed. In addition, it is desirable that anti-HCV activity be repeatedly quantitated rather than at a single time point and the animal model of HCV replication facilitates such observations.

Preferably, the reporter gene is easily assayed. For example, the reporter gene may give a detectable signal, such as a visible signal. The reporter gene may encode a protein which gives a visible signal itself, or which catalyses a reaction which gives a visible change *e.g*. a fluorescent protein or an enzyme. The reporter gene may encode an enzyme such as a beta-galactosidase or a peroxidase, both of which are commonly used with coloured substrates and/or products. The reporter gene may encode a fluorescent protein, such as green fluorescent protein (GFP) or a fluorescent derivative thereof such as YFP or CFP. The reporter gene may encode a luminescent protein, such as luciferase. The reporter gene may drive DNA replication in the cell or may encode a drug resistance marker. The reporter gene may encode an antigen that can be detected with an antibody that specifically binds the antigen. The reporter gene may also be a unique nucleic acid sequence that can be detected utilizing nucleic acid hybridization and/or amplification techniques. The above list is exemplary and is not meant to be limiting to sources for the reporter gene, many more which can readily be envisioned by the person of ordinary skill in the art.

Reporter genes that may find use in this invention include, *e.g*., antigens that have a known antibody for detection (e.g., human growth hormone and antibody to human growth hormone) and enzymes that have an activity that can be detected *in vitro* or in vivo (*e.g*., alkaline phosphatase, the luciferases, green fluorescent protein and the like). Additionally, as indicated above, detection of the presence of nucleic acid encoding the reporter can also be monitored to determine replication, expression or particle production.

Thus, as defined herein, detection of reporter gene "activity" includes the detection of the physical presence or chemical activity of the product of the reporter gene and also includes detection of all or part of a nucleic acid sequence that is specific for the reporter gene.

In some embodiments, the replicon uses the luciferase gene as a reporter. Luciferase activity can be evaluated *in vivo* in live animals according to techniques and equipment available from Xenogen, Inc. The animals, *e.g.,* mice, are injected intraperitoneally with luciferin as the substrate for luciferase. These animals are then anesthetized and the luciferase activity is quantitated by capturing the luminescent signal using a CCD camera. In this way, the animals can be repetitively measured over any given timeframe. In addition to luciferase, there are other reporter genes that can be quantitated *in vivo.* Green Fluorescence Protein, Red Fluorescence Protein or variants thereof can be visualized *in situ* using a halogen light source and CCD camera. A third method of imaging involves using HSV thymidine kinase as a reporter gene. In this method, the amount of reporter gene activity is quantitated using a microPET system. Animals are given the substrate 9-(4- [18F]Fluoro-3-hydroxymethyl butyl)guanine prior to being evaluated by microPET.

Instead of or in addition to using a reporter whose signal is detected *in vivo,* the model may have a reporter gene whose activity may be quantitated *in vitro* from a biopsy *e.g*. in an extract from a tumor after the mouse is necropsied. Reporter genes that could be used in this way include enzymes *e.g*. luciferase, β-galactosidase, peroxidase, CAT, and other similar reporters. In addition, reporter genes that encode for secreted proteins that are measurable in blood may be used. Examples of such reporter genes are genes encoding for secreted alkaline phosphatase, human growth hormone. Reporter genes can also be a sequence specific marker.

### Cell Lines

Any cell line capable of supporting HCV replicon replication in the host may be used. Besides the Huh7 cell line, the human cancer cell line HeLa, murine hepatoma Hepa1-6, HEK293human hepatoma cell lines HepG2 and IMY-N9 (a cell line derived from fusing human hepatocytes and HepG2 cells) are also known to support HCV replicon replication. Therefore, any of these tumor cell lines, or any other cell line that supports the replication of an HCV genomic or sub-genomic replicon (expressing the HCV non-structural proteins including protease and/or polymerase) may be used in the model. In some embodiments, the cell line is derived from human cells, preferably from human hepatoma cell line Huh7.

One cell line for use with the invention is derived from a hepatocellular carcinoma, namely the human hepatoma cell line known as 'Huh7' [Nakabayashi et al. (1982) Cancer Res 42:3858-63]. A useful Huh7-derived cell line is '21-5', which supports a full length HCV replicon [Pietschmann et al. (2002) J Virol 76:4008-21.]. Other suitable cell lines are Huh-7.5 and Huh-7.8, which are sub-lines of Huh-7 that can support complete HCV replication in cell culture [Blight et al. (2002) J. Virol. 76:13001-14; WO2004/044182], with Huh-7.5 being preferred. Cells derived by passaging of Huh7 cells (and their derivatives) can also be used, as well as cells derived by treating Huh7 cells with α-interferon and/or γ-interferon. As described in WO2004/044182, cell lines permissive for HCV can be prepared by a process comprising (a) culturing cells infected with HCV; (b) curing the cells of HCV; and (c) identifying a sub-line of the cured cells that is permissive for HCV replication.

The use of human-derived cells in animal models for HCV replication is desirable for several reasons. First, using human-derived cells may provide a more accurate model of HCV replication and response to treatment in humans. Second, using homologous cells may induce an interferon response from the host that inhibits the replicon, and so the cells are preferably xenogeneic with respect to the host organism. This would make it difficult to sustain the model as well as ascertain the effects of candidate compounds or protocols. For example, using replicon-harboring murine cells may cause a murine interferon-alpha response that inhibits the replicon, whereas human-derived cells are not susceptible to murine interferon-alpha.

It should be noted that in some embodiments, the cells are tumor cells. Tumor cells (*e.g*., Huh-7, HeLa, etc.) may be more easily passaged through the host. However, the invention is not limited to tumor cells but may be practiced with any cell or cell line that supports the replication of an HCV replicon.

According to various embodiments, the cell line may be adapted for 1) increased tumor growth rate *in vivo* in the host (wherein tumor cells are used) and/or 2) increased replicon expression *in vivo* in the host (for example, as measured by a bioluminescent luciferase signal where a luciferase reporter is used). Cell lines that have been adapted for increased tumor growth rate and/or adapted for stability *in vivo* in the model may be referred to herein as adapted cell lines.

According to various embodiments, the cell line may first be adapted for *in vivo* tumor growth kinetics and then adapted for increased replicon expression.

Fast Growing Adapted Cells: In some embodiments, the cell line for growth kinetics may be adapted by *in vivo* passaging through the host, typically by serial passaging.

In some embodiments, a passage involves implanting cells subcutaneously and allowing tumors to grow. The largest (*i.e.* fastest growing) tumors are identified and harvested for further passaging if applicable. The tumors are excised and minced into brei. The brei is combined for implantation and another passage. In this manner, the cells may be adapted for increased tumor growth rate in mice. The largest tumors from the final passage are harvested and expanded in cell culture to create the adapted cell line.

In some embodiments, a passage involves implanting cells in the liver and allowing tumors to grow. The fastest growing tumors are identified and harvested for further passaging if applicable as explained above. The largest tumors from the final passage are harvested and expanded in cell culture to create the adapted cell line.

Fig. 3 shows kinetics of tumor formation by 5-2 Huh7 cells and S6.1 cells. The S6.1 cell line is derived from 5-2 Huh7 cells by serial subcutaneous passaging *in vivo* in mice. (The S6.1 line is labeled "Huh7(5-2)-S6.1" as it is derived from 5-2 Huh7 cells). Generation of the S6.1 cell line is described in detail below in the Examples. Two groups of 5-2 Huh7 cells and one groups of S6.1 cells were implanted subcutaneously in SCID/bg mice. Tumor volumes were measured. Tumor formation is significantly faster in the mouse-adapted S6.1 cells, with the mean tumor volume showing a tenfold increase by day 20. This indicates that cell lines generated by serial passage *in vivo* as described have improved the tumor growth kinetics.

The S6.1 cell and cell lines in the Examples given below were derived from replicon-harboring 5-2 Huh7 cell line. However, one of skill in the art would understand that cells not harboring the replicon could be adapted for growth rate and then subsequently transfected with the replicon (for use in the model or to be adapted for increased stability).

### Stable Replicon-harboring host-Adapted Cells:

In some embodiments, stable replicon-harboring cells may be isolated by performing a luciferase (or other reporter) assay in the cells and then expanding the cells expressing the highest luciferase signal.

In embodiments wherein adaptation for growth kinetics is performed first, the cells adapted for growth kinetic may not contain the replicon (*e.g.*, the cells may have been "cured" of the replicon during passaging or the adapted cells may be derived from cells not harboring the replicon). In these cases, the cells may be transfected with the replicon and cloned (selecting the replicon-harboring cell clones by G418 selection) prior to performing the luciferase assay.

In some embodiments, isolation of a stable replicon may further include implanting (*e.g*., subcutaneously or in liver) the tumor cells expressing the highest luciferase signal in the assay described above in the host to grow a tumor. The tumor is allowed to grow for a period of time, for example about 40 days, is harvested, and then cloned in the presence of G418. Luciferase activity of the cell clones may be measured to identify a cell line or lines that have high expression of luciferase.

It should be noted that while adaptation for growth and/or replicon-stability may be desirable for some studies and cell lines, the invention may also be practiced with directly derived (non-adapted) cell lines, for example, replicon-containing 5-2 Huh7 cells.

In some embodiments, it may not be necessary or desirable to have fast growing adapted cells, for example in some embodiments slow growing adapted cells will have utility for monitoring HCV replication for longer time periods such as more than 7 days. For example, some studies, especially short-term studies, may be performed prior to significant palpable tumor growth. In some embodiments wherein tumor cells are used, the cells may be adapted and implanted in a way to maximize tumor growth. In other embodiments, it may not be desirable for cells to have significant tumor growth and animal models may be used (*e.g*. treated with test compounds) before palpable tumors become present.

### Host

The invention may be practiced using animals, preferably small animals and particularly mammals for which methods for administering or implanting cells and administering compounds to determine treatment or response are well known. In some preferred embodiments, rodents are used, and in one preferred embodiment, mice are used. Although the Examples refer to mice, given the details herein, one of skill would understand how to implement the invention with other types of rodents and other small animals. For example, suitable hosts can include, but are not limited to: a mouse; a rat; a woodchuck; and a shrew, such as a tree shrew.

The animal may be immuno-compromised by genetic mutation (*e.g*. athymic nude mice or rats, SCID mice or rats, Rag1 mice, Rag2 mice), by administration of chemical immuno-suppressants, by irradiation (*e.g*. gamma-irradiation) or a combination thereof. One class of chemical immuno-suppressants is gluco-corticoids, such as cortisone. See Jeanette I. Webster, Leonardo Tonelli, Esther M. Sternberg, "Neuroendocrine Regulation of Immunity," Annu. Rev. Immunol. 2002, 20:125-63. Other examples include azathioprine, tacrolimus, mycophenolate mofetil and cyclosporine, generally given by the intravenous or oral route. Further chemical immuno-suppressants include cyclophosphamides, azathioprines and cyclosporins. Other compounds that affect the bone marrow (such as cancer drugs) may also be used. Typically, both the innate and adaptive immune systems are compromised.

A wide range of SCID mice are available *e.g.* JAX™ mice from The Jackson Laboratory (www.jax.org). These include SCID-beige (or SCID-bg) mice, which are suitable for use with the invention.

As mentioned above, in some embodiments, human-derived cell lines are preferred. One difficulty in using human-derived (or other heterologous) cell lines in animal models is that the animal may reject the cells. Immuno-suppressing or compromising the animal may prevent the animal from rejecting cells. However, high fatality rates may result if the level of immuno-suppression is too high. Further, different cell lines may require different types of immuno-compromised hosts.

Applicants have found that gamma-irradiated SCID mice work particularly well for Huh7-derived lines. The animal is gamma-irradiated by exposure to gamma rays In one embodiments, the exposure occurs on the day of cell implantation and prior to implantation of tumor cells.

It has been found that gamma-irradiated SCID mice maintain the signal longer than SCID/bg mice. Figs. 5A-C show whole body imaging of a representative gamma-irradiated SCID mouse subcutaneously implanted with S6.1-6 cells (Fig 5A) and non-irradiated SCID/bg mice subcutaneously implanted with S6.1-6 (Fig. 5B) or 5-2 Huh7 (Fig. 5C) cells. As explained below, the S6.1-6 cell line is a 5-2 Huh7-derived cell line that is serially passaged through mice. As can be seen in Figs. 5A-C, the gamma-irradiated mouse maintains a signal for all seven days of the study, while the SCID/bg mice do not. Fig. 6A compares the mean bioluminescence over time of SCID/bg and gamma-irradiated SCID mice harboring subcutaneously implanted replicon-harboring S6.1-6 cells. Fig. 6B shows mean bioluminescence over time of SCID/bg mice harboring subcutaneously implanted replicon-harboring 5-2 Huh7 cells. The horizontal dashed line parallel to the X-axis indicates the background signal. The SCID/bg mice have a rapid decline in signal that approaches the background level of bioluminescence at around 4 days. Although the S6.1-6 cells have an improved signal over that of the 5-2 Huh7 cells in SCID/bg mice (showing slightly higher signal at day 3), the signal approaches background at around 4 days both groups of SCID/bg mice. The gamma-irradiated mice maintain an easily detectable signal above background for all 7 days of the experiment.

### Targets

In some embodiments, the target organ is the liver of the animal. This most closely resembles the human situation for which the vast majority of HCV infection is in the liver. Three surgical techniques that can be used to get the tumor cells into the liver are: 1) intrasplenic injection; 2) direct injection into the liver parenchyma; and 3) injection into the portal vein. If injected into the spleen, the cells may enter the portal circulation from intrasplenic injection and will arrest in the liver and extravasate into the liver parenchyma.

The replicon-harboring cells may also be implanted or introduced to the subcutaneous space of the animal by injection. Because subcutaneous injection is a simpler technique, this may be preferred in some embodiments where, for example, rapid screening is desired.

In general, replicons are not introduced by using human innocula from infected sera.

### Compounds

The inventors have found that the animal model described herein can be used to identify compounds that will have a synergistic or additive effect on the replication or formation or production of HCV in vivo. Compounds have been identified that are a combination of an immunomodulatory compound and another antiviral agent. In one embodiment the immunomodulatory compound is interferon-ά and the other antiviral agent is an HCV NS3/4A protease The invention thus includes combinations of immunomodulatory compounds and other antiviral agents.

An "antiviral agent" as used herein refers to an agent that is effective in reducing or inhibiting the formation and/or replication of a virus in a mammal. Anti viral agents as used herein is meant to include agents that act generally on the immune system to inhibit or reduce viral replication and also to include agents that act specifically on virus biology to reduce or inhibit viral replication. Anti viral agents can include immunomdoulatory agents such as SMIPS , interferons and the like and can also include agents targeted at interfering with one or more virus functions.

In the case of anti HCV agents, any antiviral agent that inhibits the replication or particle formation of HCV infectious virus is considered an antiviral agent and an anti-HCV virus agent. This includes agents that interfere with either host or viral mechanisms necessary for the formation and/or replication of a virus in a mammal. Anti-HCV virus agents include, for example, ribavirin, amantadine, VX-497 (merimepodib, Vertex Pharmaceuticals), VX-498 (Vertex Pharmaceuticals), Levovirin, Viramidine, Ceplene (maxamine), XTL-001 and XTL-002 (XTL Biopharmaceuticals), MN283-Valopictitabine, Idenix). Anti viral agents can also include nucleic acids (siRNA and antisense RNA, e.g.) or small molecules that may interfere with one or more enzymatic activities necessary for viral replication and packaging such as protease, helicase and polymerase functions. Anti-HCV virus agents can also include nucleic acids (siRNA and antisense RNA, e.g.) or small molecules that may interfere with one or more enzymatic activities necessary for HCV replication and packaging such as protease, helicase and polymerase functions. Such nucleic acids can be provided in the form of anti-sense molecules, double stranded duplex DNA, siRNA and the like. Antiviral agents thus include immunomodulatory compounds as described further below, either as non-specific or specific modulators of the immune system. Anti HCV agents can also include vaccines such as therapeutic and prophylactic vaccines against HCV and antibodies or small molecules which interfere with HCV receptor binding.

### Candidate compounds

Typical candidate compounds for use in all the screening methods of the invention include, but are not restricted to, peptides, peptoids, proteins, lipids, metals, small organic molecules, RNA aptamers, antibiotics and other known pharmaceuticals, polyamines, antibodies or antibody derivatives (*e.g*. antigen-binding fragments, single chain antibodies including scFvs, *etc.*), and combinations or derivatives thereof. Small organic molecules have a molecular weight of about more than 50 and less than about 2,500 daltons, and most preferably between about 300 and about 800 daltons. Candidate compounds may be derived from large libraries of synthetic or natural compounds. For instance, synthetic compound libraries are commercially available from MayBridge Chemical Co. (Revillet, Cornwall, UK) or Aldrich (Milwaukee, WI). Alternatively, libraries of natural compounds in the form of bacterial, fungal, plant and animal extracts may be used. Additionally, candidate compounds may be synthetically produced using combinatorial chemistry either as individual compounds or as mixtures. Compounds can include antibodies or fragments of antibodies that retain the antibody antigen binding capacity.

### Immunomodulatory compounds

Immunomodulatory compounds or agents (or "immunomodulators) for use in the invention include agents (compounds or biologicals) that are effective to enhance or potentiate the immune system response in a mammal and or to reduce or inhibit HCV replication and/or particle formation.

Immunomodulatory agents include, for example, class I interferons (such as α-, β-, δ- and ω-interferons,τ-interferons, consensus interferons and asialo-interferons), class II interferons (such as γ-interferons) and pegylated interferons. Other immunomodulatory compounds for use in the present invention include stimulatory molecules which improve immunogen presentation to lymphocytes, such as B7-1 or B7-2, or cytokines, lymphokines, and chemokines, including but not limited to cytokines such as IL-2, modified IL-2 (cys125 to ser125), GM-CSF, IL-12, γ-interferon, IP-10, MIP1β, FLP-3, ribavirin and RANTES, may be included in the composition.

Immunomodulators for use in the present invention can also include Small Molecule Immune Potentiators (SMIPS), nucleoside analogues and other Toll like receptor agonists as further described below.

Preferred TLR modulators are agonists of TLR7 (e.g. imidazoquinolines), TLR8 and/or TLR9 (e.g. CpG oligonucleotides).

TLR7 modulators: including loxoribine, a guanosine analogue at positions N7 and C8, isatoribine, ANA-971, ANA-975, or an imidazoquinoline compound, or derivative thereof. In one embodiment, the TLR agonist is imiquimod or resiquimod. Further TLR7 agonists are described in W002085905;

TLR8 modulators: an imidazoquinoline molecule, for example resiquimod (R848); resiquimod is also capable of recognition by TLR-7. Other TLR-8 agonists which may be used include those described in W02004071459;

TLR9 modulators: In one embodiment, the TLR agonist capable of causing a signaling response through TLR-9 is HSP90 or a DNA containing unmethylated CpG nucleotide, in particular sequence contexts described below with CpG motifs.

### Immunostimulatory oligonucleotides

Immunostimulatory oligonucleotides suitable for use as immunomodulators in the invention include nucleotide sequences containing a CpG motif (a sequence containing an unmethylated cytosine followed by guanosine and linked by a phosphate bond). Bacterial double stranded RNA or oligonucleotides containing palindromic or poly(dG) sequences have also been shown to be immunostimulatory.

The CpG's can include nucleotide modifications/analogs such as phosphorothioate modifications and can be double-stranded or single-stranded. Optionally, the guanosine may be replaced with an analog such as 2'-deoxy-7-deazaguanosine. See Kandimalla, et al., "Divergent synthetic nucleotide motif recognition pattern: design and development of potent immunomodulatory oligodeoxyribonucleotide agents with distinct cytokine induction profiles", Nucleic Acids Research (2003) 31(9): 2393-2400; WO02/26757 and WO99/62923 for examples of possible analog substitutions. The adjuvant effect of CpG oligonucleotides is further discussed in Krieg, "CpG motifs: the active ingredient in bacterial extracts?", Nature Medicine (2003) 9(7): 831-835; McCluskie, et al., "Parenteral and mucosal prime-boost immunization strategies in mice with hepatitis B surface antigen and CpG DNA", FEMS Immunology and Medical Microbiology (2002) 32:179-185; WO98/40100; US Patent No. 6,207,646; US Patent No. 6,239,116 and US Patent No. 6,429,199.

The CpG sequence may be directed to TLR9, such as the motif GTCGTT or TTCGTT. See Kandimalla, et al., "Toll-like receptor 9: modulation of recognition and cytokine induction by novel synthetic CpG DNAs", Biochemical Society Transactions (2003) 31 (part 3): 654-658. The CpG sequence may be specific for inducing a Thl immune response, such as a CpG-A ODN, or it may be more specific for inducing a B cell response, such a CpG-B ODN. CpG-A and CpG-B ODNs are discussed in Blackwell, et al., "CpG-A-Induced Monocyte IFN-gamma-Inducible Protein-10 Production is Regulated by Plasmacytoid Dendritic Cell Derived IFN-alpha", J. Immunol. (2003) 170(8):4061-4068; Krieg, "From A to Z on CpG", TRENDS in Immunology (2002) 23(2): 64-65 and WO01/95935. Preferably, the CpG is a CpG-A ODN.

Examples of CpG nucleotides include the following sequences, which may contain phosphorothioate modified internucleotide linkages:

TCC ATG ACG TTC CTG ACG TT (CpG 1826); TCT CCC AGC GTG CGC CAT
(CpG 1758); ACC GAT GAC GTC GCC GGT GAC GGC ACC ACG; TCG TCG
TTT TGT CGT TTT GTC GTT (CpG 2006); and TCC ATG ACG TTC CTG ATG
CT (CpG 1668). See WO 05/25614.

Preferably, the CpG oligonucleotide is constructed so that the 5' end is accessible for receptor recognition. Optionally, two CpG oligonucleotide sequences may be attached at their 3' ends to form "immunomers". See, for example, Kandimalla, et al., "Secondary structures in CpG oligonucleotides affect immunostimulatory activity", BBRC (2003) 306:948-953; Kandimalla, et al., "Toll-like receptor 9: modulation of recognition and cytokine induction by novel synthetic GpG DNAs", Biochemical Society Transactions (2003) 31(part 3):664-658; Bhagat et al., "CpG penta- and hexadeoxyribonucleotides as potent immunomodulatory agents" BBRC (2003) 300:853-861 and WO03/035836.

### Small Molecule Immunopontentiators (SMIPs)

### 1. Imidazoquinoline Compounds.

Examples of imidazoquinoline compounds suitable for use adjuvants in the invention include Imiquimod and its analogues, described further in Stanley, "Imiquimod and the imidazoquinolines: mechanism of action and therapeutic potential" Clin Exp Dermatol (2002) 27(7):571-577; Jones, "Resiquimod 3M", Curr Opin Investig Drugs (2003) 4(2):214-218; Wu et al. (2004) Antiviral Res. 64(2):79-83 Vasilakos et al. (2000) Cell Immunol. 204(1):64-74 US patents 4689338, 4929624, 5238944, 5266575, 5268376, 5346905, 5352784, 5389640, 5395937, 5482936, 5494916, 5525612, 6083505, 6440992, 6627640, 6656938, 6660735, 6660747, 6664260, 6664264, 6664265, 6667312, 6670372, 6677347, 6677348, 6677349, 6683088, 6703402, 6743920, 6800624, 6809203, 6888000 and 6924293.

Preferred SMIPs include:
N2-methyl-1-(2-methylpropyl)-1H-imidazo[4,5-c]quinoline-2,4-diamine;
N2,N2-dimethyl-1-(2-methylpropyl)-1H-imidazo[4,5-c]quinoline-2,4-diamine;
N2-ethyl-N2-methyl-1-(2-methylpropyl)-1H-imidazo[4,5-c]quinoline-2,4-diamine;
N2-methyl-1-(2-methylpropyl)-N2-propyl-1H-imidazo[4,5-c] quinoline-2,4-diamine;
1-(2-methylpropyl)-N2-propyl-1H-imidazo[4,5-c]quinoline-2,4-diamine;
N2-butyl-1-(2-methylpropyl)-1H-imidazo[4,5-c]quinoline-2,4-diamine;
N2-butyl-N2-methyl-1-(2-methylpropyl)-1H-imidazo[4,5-c]quinoline-2,4-diamine;
N2-methyl-1-(2-methylpropyl)-N2-pentyl-1H-imidazo[4,5-c]quinoline-2,4-diamine;
N2-methyl-1-(2-methylpropyl)-N2-prop-2-enyl-1H-imidazo[4,5-c]quinoline-2,4-diamine;
1-(2-methylpropyl)-2-[(phenylmethyl)thio]-1H-imidazo[4,5-c]quinolin-4-amine;
1-(2-methylpropyl)-2-(propylthio)-1H-imidazo[4,5-c]quinolin-4-amine;
2-[[4-amino-1-(2-methylpropyl)-1H-imidazo[4,5-c]quinolin-2-yl](methyl)amino]ethanol;
2-[[4-amino-1-(2-methylpropyl)-1H-imidazo[4,5-c]quinolin-2-yl](methyl)amino]ethyl acetate;
4-amino-1-(2-methylpropyl)-1,3-dihydro-2H-imidazo[4,5-c]quinolin-2-one;
N2-butyl-1-(2-methylpropyl)-N4,N4-bis(phenylmethyl)-1H-imidazo[4,5-c]quinoline-2,4-diamine;
N2-butyl-N2-methyl-1-(2-methylpropyl)-N4,N4-bis(phenylmethyl)-1H-imidazo[4,5-c]quinoline-2,4-diamine;
N2-methyl-1-(2-methylpropyl)-N4,N4-bis(phenylmethyl)-1H-imidazo[4,5-c]quinolme-2,4-diamine;
N2,N2-dimethyl-1-(2-methylpropyl)-N4,N4-bis(phenylmethyl)-1H-imidazo[4,5-c]quinoline-2,4-diamine;
1-{4-amino-2-[methyl(propyl)amino]-1H-imidazo[4,5-c]quinolin-1-yl}-2-methylpropan-2-ol;
1-[4-amino-2-(propylamino)-1H-imidazo[4,5-c]quinolin-1-yl]-2-methylpropan-2-ol;
N4,N4-dibenzyl-1-(2-methoxy-2-methylpropyl)-N2-propyl-1H-imidazo[4,5-c]quinoline-2,4-diamine.

*Nucleoside Analogs.*

A nucleoside analog, such as: (a) Isatorabine (ANA-245; 7-thia-8-oxoguanosine): 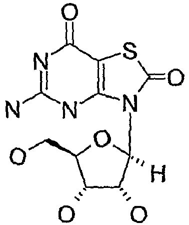 and prodrugs thereof; (b)ANA975; (c) ANA-025-1; (d) ANA380; (e) the compounds disclosed in references iv US 6,924,271 to US2005/0070556vi US 5,658,731; (f) a compound having the formula: 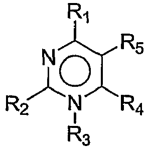 wherein:
R₁ and R₂ are each independently H, halo, -NRₐR_{b}, -OH, C₁₋₆ alkoxy, substituted C₁₋₆ alkoxy, heterocyclyl, substituted heterocyclyl, C₆₋₁₀ aryl, substituted C₆₋₁₀ aryl, C₁₋₆ alkyl, or substituted C₁₋₆ alkyl;
R₃ is absent, H, C₁₋₆ alkyl, substituted C₁₋₆ alkyl, C₆₋₁₀ aryl, substituted C₆₋₁₀ aryl, heterocyclyl, or substituted heterocyclyl;
R₄ and R₅ are each independently H, halo, heterocyclyl, substituted heterocyclyl, -C(O)-R_{d}, C₁₋₆ alkyl, substituted C₁₋₆ alkyl, or bound together to form a 5 membered ring as in R₄₋₅: 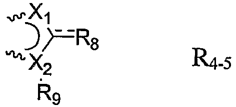 the binding being achieved at the bonds indicated by a 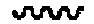
X₁ and X₂ are each independently N, C, O, or S; R₈ is H, halo, -OH, C₁₋₆ alkyl, C₂₋₆ alkenyl, C₂₋₆ alkynyl, -OH, -NRₐR_{b}, -(CH₂)ₙ-O-R_{c}, -O-(C₁₋₆ alkyl), -S(O)ₚRₑ, or -C(O)-R_{d};
R₉ is H, C₁₋₆ alkyl, substituted C₁₋₆ alkyl, heterocyclyl, substituted heterocyclyl or R₉ₐ, wherein R₉ₐ is: 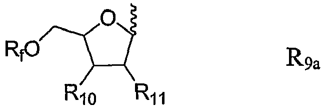 the binding being achieved at the bond indicated by a 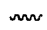
R₁₀ and R₁₁ are each independently H, halo, C₁₋₆ alkoxy, substituted C₁₋₆ alkoxy, -NRₐR_{b}, or -OH;
each Rₐ and R_{b} is independently H, C₁₋₆ alkyl, substituted C₁₋₆ alkyl, - C(O)R_{d}, C₆₋₁₀ aryl;
each R_{c} is independently H, phosphate, diphosphate, triphosphate, C₁₋₆ alkyl, or substituted C₁₋₆ alkyl;
each R_{d} is independently H, halo, C₁₋₆ alkyl, substituted C₁₋₆ alkyl, C₁₋₆ alkoxy, substituted C₁₋₆ alkoxy, -NH₂, -NH(C₁₋₆ alkyl), - NH(substituted C₁₋₆ alkyl), -N(C₁₋₆ alkyl)₂, -N(substituted C₁₋₆ alkyl)₂, C₆₋₁₀ aryl, or heterocyclyl;
each Rₑ is independently H, C₁₋₆ alkyl, substituted C₁₋₆ alkyl, C₆₋₁₀ aryl, substituted C₆₋₁₀ aryl, heterocyclyl, or substituted heterocyclyl;
each R_{f} is independently H, C₁₋₆ alkyl, substituted C₁₋₆ alkyl, -C(O)R_{d}, phosphate, diphosphate, or triphosphate;
each n is independently 0, 1, 2, or 3;
each p is independently 0, 1, or 2; or
or (g) a pharmaceutically acceptable salt of any of (a) to (f), a tautomer of any of (a) to (f), or a pharmaceutically acceptable salt of the tautomer; Loxoribine (7-allyl-8-oxoguanosine) [US patent 5,011,828].

### 3. Thiosemicarbazone Compounds.

Examples of thiosemicarbazone compounds, as well as methods of formulating, manufacturing, and screening for compounds all suitable for use as adjuvants in the invention include those described in WO04/60308. The thiosemicarbazones are particularly effective in the stimulation of human peripheral blood mononuclear cells for the production of cytokines, such as TNF- .

### 4. Tryptanthrin Compounds.

Examples of tryptanthrin compounds, as well as methods of formulating, manufacturing, and screening for compounds all suitable for use as adjuvants in the invention include those described in WO04/64759. The tryptanthrin compounds are particularly effective in the stimulation of human peripheral blood mononuclear cells for the production of cytokines, such as TNF- *.*

### 5. Additional SMIPs

(i) Compounds disclosed in reference WO2004/87153, including: Acylpiperazine compounds, Indoledione compounds, Tetrahydraisoquinoline (THIQ) compounds, Benzocyclodione compounds, Aminoazavinyl compounds, Aminobenzimidazole quinolinone (ABIQ) compounds [US 6,605,617,vii WO02/18383], Hydrapthalamide compounds, Benzophenone compounds, Isoxazole compounds, Sterol compounds, Quinazilinone compounds, Pyrrole compounds [WO2004/018455], Anthraquinone compounds, Quinoxaline compounds, Triazine compounds, Pyrazalopyrimidine compounds, and Benzazole compounds [WO03/082272].
(ii) Methyl inosine 5'-monophosphate ("MIMP") [Signorelli & Hadden (2003) Int Immunopharmacol 3(8):1177-86.].
(iii) A polyhydroxlated pyrrolizidine compound [WO2004/064715], such as one having formula: 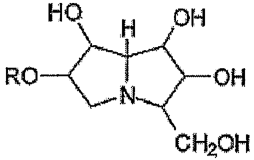 where R is selected from the group comprising hydrogen, straight or branched, unsubstituted or substituted, saturated or unsaturated acyl, alkyl (*e.g*. cycloalkyl), alkenyl, alkynyl and aryl groups, or a pharmaceutically acceptable salt or derivative thereof. Examples include, but are not limited to: casuarine, casuarine-6-α-D-glucopyranose, 3-*epi-*casuarine, 7-*epi*-casuarine, 3,7-di*epi*-casuarine, *etc.*
(iv) A gamma inulin [Cooper (1995) Pharm Biotechnol 6:559-80] or derivative thereof, such as algammulin.

### PK/PD studies

The animal models of the present invention may be used for pharmacokinetic, pharmacodynamic and toxicology studies of potential anti-viral compounds and protocols. The model may also be used to monitor the course of HCV replication and also to determine the drug mechanism of action.

The animal models may be used to identify and evaluate compounds or protocols that inhibit replicon replication. The animal models may also be used to identify anti-viral compounds that may inhibit other steps in the viral life cycle (*e.g*., compounds that inhibit virus packaging or egress from the cell).

Animals are treated with a candidate compound or using a candidate protocol. Alternatively, animals are treated with a combination of compounds already known or determined to be effective immunomodulators against HCV, such as, e.g., interferon-alpha Anti-viral activity may be quantified and compared with control or untreated models, or quantified and used to determine the relative rankings of multiple candidates. In some embodiments, preventative compounds or protocols are similarly identified, evaluated and/or ranked by administering the compound or protocol prior to cell implantation. In these cases, the compound or protocol could also be administered after cells are implanted.

Test agents (candidate compounds) can be administered intraperitoneally, subcutaneously, intravenously or orally. The route of administration and schedule of administration may be determined individually for each candidate compound and may be based upon the amount of drug exposure generated for each delivery method. Drug exposure is measured in pharmacokinetic studies. By knowing how long the test compound remains in the body, this information can be used to determine the administration schedule of multiple doses of test compound. The schedule may be chosen to maintain a therapeutically effective amount of test compound over a period of several days.

Anti-viral activity may be measured in several ways. A first method is using one or more of the reporter genes mentioned above (*e.g*. a luciferase assay technique). Reporter gene activity, or the amount of the reporter protein should correlate with the amount of HCV replicon replication. In addition to measuring the level of reporter protein, the efficacy of the candidate compound or protocol may be determined by measuring the level of HCV RNA or the level of the viral proteins. Dose and/or time-dependent response curves (e.g., EC₅₀, IC₅₀) may be determined for candidate compounds and protocols.

In some embodiments, a second reporter gene may be stably or transiently transfected into the cell to allow the viability of cells to be assessed. If a test compound kills target cells then expression of the first reporter will not be seen, but because of cell death rather than because of any antiviral activity. Thus the second reporter acts as a marker for viability of cells containing the HCV replicon.

One suitable second reporter is a *Renilla* luciferase gene. Another method to determine this is to quantitate the gene copy number of an HCV gene on the sub-genomic replicon, and a human gene contained in the Huh7 cells. The replicon content per cell remains relatively stable during Huh7 cell growth *in vitro* and this is believed to be the case *in vivo.* Thus, by comparing the ratio of HCV gene copy number to human gene copy number in tumor tissue, it can be determined if treatment of mice with an HCV inhibitor is inhibiting replication of the sub-genomic replicon (HCV gene to Human gene copy number ratio decreases) or if the compound is inhibiting human tumor cell growth or inducing tumor cell death of the cells harboring the replicon (HCV to Human gene copy number ratio stays relatively constant). To accomplish this, the animal would be necropsied and tumor samples taken for molecular analysis. HCV gene and human gene copy number per mg of tumor tissue would quantitated at day zero for the baseline and again at a later time or times. However, measurements that may be performed *in vivo* are preferred.

In addition to the efficacy information determined as discussed above, the model may be used to obtain initial pharmacokinetic (concentration over time) and/or toxicology data. For example, margin of safety, lowest effective dose and the lowest dose exhibiting toxic signs may be determined. Pharmacokinetics and toxicology quantifications are well known and may be determined by any known method. Examples of such methods are described in Y. Kwon Handbook of Essential Pharmacokinetics, Pharmacodynamics and Drug Metabolism for Industrial Scientists, Klewer Academic/Plenum Publishers, New York, 2001; R.D. Schoenwald (editor) Pharmacokinetics in Drug Discovery and Development, CRC Press, 2002; and S.C. Gad, Drug Safety Evaluation, Wiley-Interscience, 2002.

Although implantation of the replicon-containing cells may not be necessary for pharmacokinetics or toxicology studies, the model provides the advantage of allowing these studies to be carried out simultaneously with and in the same animals as pharmacodynamic studies.

According to various embodiments, the models of the present invention may be used for studies of different lengths.

In one embodiment, the model may be used for relatively short-term studies. Short-term studies may be under 10 days (from test compound treatment), and can be as short as 2-7 or even fewer days. For example, as shown below in Example 7, treatment of HCV animal models according to an embodiment of the invention with a known anti-viral compound (IFN-alpha) resulted in a detectable drop in mean bioluminescence at day 2 as compared to an untreated model.

As used herein, "interferon" or interferon-a" or "interferon alpha" or interferon-α " or any of the above in combnination with "2B" are meant to indicate interferon alpha 2B.

In another embodiment, the model may be used for longer-term studies. Longer term studies may be especially useful for evaluation of toxicity or safety margin (a measure of activity versus toxicity) of compounds or therapies. Toxic effects may increase with exposure to a compound over time. Also, drug resistance can be assessed since mutations that permit a compound to be rendered ineffective could be selected for after repeated administration of test compounds and long term treatment with a compound may induce such mutations. A long term model not only allows for evaluation of PK/PD of a compound, but also for improved evaluation of toxicity and the potential development of drug resistance.

In another embodiment, the invention further provides a tumor cell line made by the following process: tumor cells harboring a replicon as described herein are implanted subcutaneously in SCID mice, which are irradiated before cell implantation (Other immuno compromised mechanisms can be employed);
excising a tumor emitting strong bioluminescence;
culturing in vitro the bioluminescent portion of the tumor in the presence of a selective marker for the replicon;
selecting a cell colony with high luciferase expression and growing the resultant clone that grows in the presence of the positive selector for the replicon (e.g., G415 as described herein). The process can be repeated one or more times to further select for a cell line with decreased sensitivity to IFN-α. The cell line can be cured of the replicon by culturing in high does interferon alpha s described herein.

As known in the art, reporters and additional methods of immunocompromising the animal can be employed.

### Examples

The following examples provide details illustrating aspects of the present invention. These examples are provided to exemplify and more clearly illustrate these aspects of the invention and are in no way intended to be limiting.

### Example 1: Plasmid construction and RNA translation and transfection efficiency

### Plasmid construction:

The replicon p114/ET (described as PILucUbiNeoNS3-3'/ET or PVI replicon as shown in Fig. 1) was constructed by deleting the 1.6kb of XbaI-NotI fragment from pFKI341PILucNS3-3'/ET **(**Lohmann, V., S. Hoffmann, U. Herian, F. Penin, and R. Bartenschlager. 2003: J Virol 77:3007-19) and replacing that fragment with the 2.6kb ofXbaI-NotI fragment from pFKI389LucUbiNeoNS3-3'/5.1. Krieger, N., V. Lohmann, and R. Bartenschlager. J Virol 75:4614-24).

The HCVI replicon is described in 2001, Journal of Virology, 75(10): 4614-4624].

RNA transfection: All the plasmids were linearized with *Sca*I, and RNA was synthesized with the MEGAscript kit (Ambion). In vitro-transcribed RNA was purified as previously described (Guo, J. T., V. V. Bichko, and C. Seeger. 2001. Effect of alpha interferon on the hepatitis C virus replicon. J. Virol. 75:8516-8523). The conditions used for the transfection of cells with total RNA were identical to those used for the transfection with in vitro-transcribed RNA (See Guo et al., referred to above). Subconfluent Huh7 cells were trypsinized and washed once with complete DMEM and once with serum-free DMEM-F12 medium. Cell pellets were resuspended in serum-free DMEM-F12 medium at a density of 10 cells/ml. To 200 ml of the cell suspensions in an electroporation cuvette (0.2-cm gap; BTX, San Diego, Calif.) 1 to 10 mg of in vitro-transcribed RNA was added. The cells were immediately electroporated with an ECM 630 apparatus (BTX) set to 200 V and 1,000 mF. After electroporation the cell suspension was kept for 5 min at room temperature and then diluted into DMEM supplemented with 10% fetal bovine serum and nonessential amino acids and seeded into a 10-cmdiameter petri dish. After 24 h, G418 was added to obtain a final concentration of 0.5 mg/ml, and medium was changed every other day. G418-resistant colonies became visible after 2 to 3 weeks.

Efficiency of translation and transfection of HCV replicons: Huh-7 cells were transfected (see above) with *in vitro* transcripts from either p114/ET (PVI replicon in Fig. 1) or the HCVI replicon (see Fig. 1) and grown using standard tissue culture techniques in the presence of G418 in order to select replicon containing, G418 resistant cells. Luciferase expression of the PVI replicon was higher than that of the HCVI replicon based on the magnitude of the RLU per 10,000 cells (see Fig. 2A). The efficiency of colony formation is also higher for the PVI replicon than the HCVI replicon. Fig. 2B shows that the number of G418 resistant colonies in Huh-7 cultures transfected with p114/ET (PILucUbiNeoNS3-3'/ET) RNA was higher than the number of colonies observed after transfection with HCVI replicon RNA even though the amount of RNA used to transfect the Huh-7 cells was equal for both transfections.

### Example 2: Generation of mouse-adapted S6.1 cells

5-2 Huh7 cells were expanded in DMEM with high glucose, 10% fetal bovine serum, L-glutamine, non-essential amino acids and 250 µg/ml G418. Cells were split in a range of 1:3 to 1:10 every 3-7 days. Five million cells in 0.2 mls HBSS were implanted subcutaneously into the right flanks of 10 female SCID-beige mice (Charles River Laboratories, Wilmington, MA). Tumor volumes (0.5 x Width x Width x Length) were measured two times per week from day 5 through day 36. Width and length of the tumor were measured with calipers. Mice were imaged for luciferase activity by *in vivo* bioluminescent imaging using the IVIS camera system (Xenogen, Alameda, CA) on days 1, 8 and 37. On day 37, only the 3 mice with the largest tumors were imaged. These 3 largest tumors (393, 468, and 532 mm³, measured on day 36) were harvested on day 37 and mashed into brei with the frosted ends of two sterilized glass slides. A 50 - 100 µl portion of brei was inserted subcutaneously with a trocar into each of 10 anesthetized SCID-beige mice. Tumor volumes in this second study were measured two times per week from day 8 through day 26. On day 28, the four largest tumors (759, 831, 1083 and 1585 mm³ as of day 26) were harvested, combined into brei and implanted as described above into another 10 SCID-beige mice. Tumor volumes were measured two times per week from day 7 through day 21. On day 22, the 3 largest tumors (584, 638 and 707 mm³ on day 21) were harvested and implanted in the same manner into 5 SCID-beige. Tumor volumes were measured two times per week from day 6 through day 23. On day 24, 3 of the larger tumors (978, 1249, and 1519 mm³ day 23) were harvested and expanded in cell culture to create the cell line Huh7(5-2)-S6.1. As shown in Fig 3, S6.1 cells form tumors more quickly than 5-2 cells when the same number of cells are implanted into mice subcutaneously.

### Example 3: Generation ofReplicon-harboring cell line S6.1-6

10ug of total RNA extracted from Huh7-114ET-7 cells (Huh7 cells harboring p114/ET , alternatively called PILucUbiNeoNS3-3'/ET or PVI in Fig. 1) was transfected into S6.1 cells following the protocol described above. Replicon-harboring cell clones were obtained by selection in the presence of G418 at the concentration of 0.5mg/ml. Luciferase assay was performed in each cell clones. Fig. 4 shows relative units of luciferase activity for each of the clones. The S6.1-6 cells expressed high level of luciferase signal and were further expanded and used for *in vivo* animal studies.

### Example 4: Generation of pooled mouse adapted cell line S6.1-P1

Mouse adapted S6.1 cells were transfected with *in vitro* transcribed RNA from linearized PVI replicon by electroporation as described above. Colonies were selected with G418 at a concentration of 0.5mg/ml. The luciferase activity was measured by Promega Glo-steady Luc assay kit as described by manufacturer. Fig. 4 shows the relative light units (RLU) of luciferase activity per 1,000 cells plotted against different cell clones. The cell clones #1,2,4,6, F and J were pooled to generate the cell line S6.1-P1.

### Example 5: Isolation of Replicon-harboring cell line S6.1-T12-2, S6.1-T15, S6.1-T7

The tumor from mouse number 12 implanted with replicon-harboring pooled S6.1 cells was harvested on day 43 and cultured in the complete DMEM medium for a week. The cells were trypsinized and then serially diluted onto 100mm plates in the presence of G418 (250ug/ml). Two weeks later the G418 resistant colonies in 100mm plates were imaged using the IVIS camera system as described above. The luciferase positive colonies were transferred to 96 well plates and expanded to measure luciferase activity. One of the luciferase expressing cell clones was named S6.1-T12-2. Other cell lines that have stable expression of Luc but form tumors more quickly than S6.1-T12-2, such as S6.1-T15 and S6.1-T7, can be made in a similar fashion.

### Example 6: Generation of mouse liver-adapted cells

5-2 Huh7 cells were seeded into the livers of 9 SCID mice (non-irradiated) by intrasplenic injection. The spleen was exteriorized and 2.5x10^5 to 3.5x10^6 cells in 0.05 mls HBSS was injected into the spleen. The spleen was placed between sterile gauze saturated with saline for 10 minutes to allow the cells to travel to the liver. Then blood vessels attached to the spleen were cauterized and the spleen detached by ligation. Mice were imaged with the Xenogen camera system on days 0, 1, and 2 and the signal dropped to background levels by day 2. Mice were again imaged on days 7, 10, 15 and 29 and no signal above background was observed. Starting at 6 weeks after cell inoculation necropsy was performed periodically to check for tumor growth in the liver. On day 48, one tumor was harvested from the liver of one mouse and cultured in vitro to create the cell line Huh7(5-2)-H2.

These Huh7(5-2)-H2 cells were then implanted directly into the livers of 10 non-irradiated SCID-bg mice. During surgery, the liver was exteriorized and two million cells (in 100 microliters of HBSS) were injected directly into a single lobe of the liver. The cells were injected into the parenchyma in two different locations of the same lobe (50 µl in each location). The surgical incision was then closed and the mice were allowed to recover from surgery overnight. Thirty days after cell inoculation, necropsy was performed on two mice to check for tumor growth in the liver. One tumor was harvested from the liver of one mouse and cultured in vitro to create the cell line Huh7(5-2)-H2L1.1. On days 34 and 40 tumors were harvested from 4 more mice to create the two cell lines Huh7(5-2)-H2L2.2 and Huh7(5-2)-H2L2.3.

The Huh7(5-2)-H2L1.1 cells were then implanted directly into the livers of another 10 non-irradiated SCID-bg mice following the same procedure described above. Five out of 10 mice first started showing distended abdomens due to tumor burden 27 days after inoculation. On this day, tumors were harvested from the livers of each of 3 of the mice. These tumors were combined as tumor brei and cultured in vitro to create the cell line Huh7(5-2)-H2L2.1. Twenty nine days after cell inoculation, tumors were harvested from the livers of another set of 3 mice. These tumors were combined as tumor brei and cultured in vitro to create the cell line Huh7(5-2)-H2L2.2

### Example 7: Bioluminescent signal stability of subcutaneously implanted 5-2 Huh 7 and S6.1-6 cells in SCID/bg and gamma-irradiated mice

S6.1-6 cells were prepared as described above. 5x10⁶ cells were subcutaneously implanted in gamma-irradiated SCID mice. 5x10⁶ 5-2 Huh7 cells (in matrigel) were subcutaneously implanted in SCID/bg (no irradiation) and 5x10⁶ 5-2 Huh7 cells (no matrigel) were subcutaneously implanted in gamma-irradiated SCID mice. The SCID mice were irradiated at 3 Gray's (GY) (∼3.2 minutes) the day of but prior to cell implantation. Cells were implanted by injection into the right flank. Matrigel was added to 5-2 Huh-7 cells in an attempt to improve the maintenance of the bioluminescent signal which was dropping off quickly in SCID/Bg mice.

*In vivo* images were taken and bioluminescence was quantified at days 1, 2 3, 4 and 7, with the exception of SCID/bg mice that received 5-2 Huh7 cells who were imaged on days 1, 3 and 4 only. Fig. 5 shows day 1, 3, 4 and 7 images of a gamma-irradiated SCID mouse and a SCID/bg mouse with S6.1-6 cells (Fig. 5A and 5B, respectively) and a SCID/bg mouse with 5-2 Huh7 cells (Fig. 5C). The bioluminescent signal is maintained (indicating that the luciferase in the replicon is being expressed) for gamma-irradiated SCID mice with S6.1 cells for seven days while the signal is only detectable in the SCID/bg mouse with 5-2 Huh7 or for S6.1-6 cells for 3 days. Figs. 6A and B are graphs showing the mean bioluminescence (of all mice) over time. The bioluminescence is essentially background on day 4 for SCID/bg mice with either 5-2 Huh7 (Fig. 6B) or for S6.1-6 cells (Fig. 6A) while the irradiated SCID mice maintain a signal recognizable over background for at least 7 days (Fig. 6A).

### Example 8: Bioluminescence of 5-2 Huh7 and S6.1-6 in a Subcutaneous model in Irradiated SCID mice

S6.1-6 cells were prepared as described above. 5x10⁶ cells were subcutaneously implanted in gamma-irradiated SCID mice. 5x10⁶ 5-2 Huh7 cells were subcutaneously implanted in gamma-irradiated SCID mice. Cells were implanted by injection (5x10⁶ cells in 0.2 ml of 100 µµg/ml human serum albumin in HBSS) into the right flank. The SCID mice were irradiated at 3 GY (∼3.2 minutes) the day of and prior to cell implantation.

Bioluminescence was quantified at days 1, 2, 3, 4, 7, 10, 17 and 21. Fig. 7 is a graph showing the mean bioluminescence (of all mice) over time. The S6.1-6 mice had a stronger signal than the 5-2 mice up to 17 days post implantation. The luciferase signal from the S6.1-6 cells increased over the first 5 days post-implantation while the signal from 5-2 cells declined over the same period of time. The data suggests that the S6.1 cells are more robust during the first 5 days post implantation. The data also shows that a signal recognizable above background (1.0 x 10⁵ photons/sec) was maintained for at least 21 days for both S6.1 and 5-2 Huh7 cells.

Tumor volume measurements of the two groups were taken on days 11, 14, 17 and 21. The mean tumor volume on day 11 was 100 mm³ for the 5-2 Huh7 model and 100mm³ for the S6.1-6 model. By day 21, it was 170 mm³ for the 5-2 Huh7 model and 570 mm³ for the S6.1-6 model.

### Example 9: Treatment of subcutaneous model with Interferon-alpha

Replicon-harboring S6.1-6 cells were prepared as described above. Female SCID mice were gamma-irradiated at 3 GY for approximately 3.2 minutes on the day of and prior to cell implantation. 5x10⁶ cells in 0.2 ml of vehicle were implanted subcutaneously into the right flanks of 42 mice, divided into three groups (control group, a low IFN-alpha dose group and a high IFN-alpha dose group). Each group contained 14 mice. Doses were given to each group as follows:
Control group: Vehicle (0.1 ml of 100 µµg/ml human serum albumin in HBSS) per day SC
Low IFN-alpha: 2,400 IU per day per 18 gram mouse IFN-alpha SC
High IFN-alpha: 200,000 IU per day per 18 gram mouse IFN-alpha SC

Interferon-alpha injection solution INTRON (Schering) was used. 18 million international units (IU) per vial; 3 million IU per 0.5 ml (NDC 0085-1168-01) Human Serum Albumin at 100ug/ml (Cat# A5843, Sigma, low endotoxin) was used as a carrier for the dilute IFN solutions. Dosing solutions were made fresh daily except weekends. *In vivo* images were taken on days 1, 2, 3, 4 and 7 before each dose. The day 1 image was used as the baseline image.

Fig. 8 shows day 1, 2, 3, 4 and 7 images of a representative mice from each of the groups: mouse #1-3 (control group), mouse #2-3 (low IFN-alpha dose) and mouse #3-5 (high IFN-alpha dose). Mean bioluminescence per day is shown in Fig. 9.

A significant drop in mean bioluminescence was detected one day (day 2) after the first dose of Inteferon-alpha at both low and high dose levels. A 3.6 fold drop in signal was seen in the low dose group and a 14 fold drop was seen in the high dose group as compared to vehicle. The treated groups continued to lose signal on days 3 and 4 and reached background levels on day 7, which was approximately 10 fold lower than the vehicle control group. The vehicle group maintained signal of 5x10^5 to 1x10^6 photons through day 27.

The time and dose-dependent antiviral effect of IFN-alpha found agrees with human clinical outcomes and the chimeric mouse xenograft model reported by Mercer et al. Nature Medicine 2001 7:927-933.

Tumor volume measurements of the vehicle group were taken from day 17 through day 27 (and through day 43 for 3 to 6 mice). The mean tumor volume on day 17 was 100mm3 and on day 27 it was 520mm3.

### Example 10: Treatment of subcutaneous tumor model with Interferon-alpha

Replicon-containing 5-2 Huh7 cells were prepared as described in Example 1 and implanted subcutaneously into SCID mice. The mice were gamma-irradiated at 3 GY for approximately 3.2 minutes on the day of and prior to implantation. Tumors were allowed to grow in the mice. On days 42 and 43, two mice were treated with vehicle and two with IFN-alpha as follows:
Control group: Vehicle (0.1 ml of 100 µµg/ml human serum albumin in HBSS) SC, QD
IFN group: 200,000 IU per day per 18 gram mouse IFN-alpha SC

Interferon-alpha injection solution INTRON (Schering) was used. 18 million international units (IU) per vial; 3 million IU per 0.5 ml (NDC 0085-1168-01) Human Serum Albumin at 100ug/ml (Cat# A5843, Sigma, low endotoxin) was used. *In vivo* images were taken on days 42 and 43 before each dose and bioluminescence was quantified.

Fig. 10 shows bioluminescence for the treated and untreated (vehicle) mice. Background signal is 1x10⁵ photons/sec. Bioluminescence decreased about 7 - 8 fold for the IFN-alpha treated mice, and less than about 1.5 fold for the untreated mice. While the mean bioluminescent signal was approximately equal for the two groups prior to dosing (1.07E+06 photons/sec for the treated group and 1.10E+06 photons/sec for the untreated group), the bioluminescence decreased dramatically for the treated group and increased slightly for the untreated group (1.35E+05 photons/sec for the treated group and 1.37E+06 photons/sec for the untreated group).

### Example 11: Treatment of Liver Model with Interferon-alpha

Replicon-containing S6.1-6 cells were prepared as described above in Example 3. The cells were harvested and re-suspended in HBSS at 1x10^6 cells/0.05 mls/injection. Female C.B17 SCID mice were shaved, gamma-irradiated at 3 GY for approximately 3.2 minutes. Mice were irradiated approximately 4 hours prior to surgery.

An incision was made through the skin and the peritoneal cavity, and then one liver lobe exposed gently. Tumor cells in 0.05 mls of HBSS were injected into the lobe at two sites (1x10^6 cells/0.05 mls/injection). The surgical incision was closed in the mice were allowed to recover from surgery overnight.

Mice were then randomized and either interferon-alpha or placebo was administered subcutaneously once a day beginning on day 1 and ending on day 7. Each group contained nine mice and the treatments were:
Group 1: Vehicle (0.1 ml of 100 ug/ml Human Serum Albumin in HBSS) SC, QD
Group 2: 200,000 IU per day per 18 gram mouse IFN-alpha 2b SC

Interferon-alpha injection solution INTRON (Schering) was used. 18 million international units (IU) per vial; 3 million IU per 0.5 ml (NDC 0085-1168-01) Dosing solutions were made fresh daily except weekends.

*In vivo* images were taken from the ventral view on days 1 (one day post-surgery), 2, 4 and 7. The day 1 image, taken before compound administration began, was used as the baseline image. The bioluminescent signal from each mouse was measured from the ventral view by injecting the mouse intraperitoneally with 150 mg/kg of the luciferase substrate luciferin, then placing the mouse and a light tight chamber and capturing the signal with a cooled CCD camera (IVIS system, Xenogen, Alameda, California). A region of interest was created around the liver area of the mouse in the signal was quantified in photons per second.

Mean bioluminescence per day is shown in Fig. 11. A significant drop in mean bioluminescence was detected one day (day 2) after the first dose of Inteferon-alpha. A one log drop in signal was seen in the treated group as compared to vehicle. By day 4, the mean luciferase signal of the treated group was after near background levels and approximately 1.5 log lower than the vehicle group.

The time-dependent antiviral effect of IFN-alpha found is consistent with human clinical outcomes.

### Examples 12: Subcutaneous Dual Reporter Model treatment with Interferon-alpha

In order to demonstrate that the reduction in bioluminescence after treatment with IFN-alpha was not due to killing the S6.1-6 replicon cells expressing firefly luciferase, a control plasmid expressing *Renilla* luciferase driven by the CMV promoter was introduced into replicon cells by transfection. Co-transfected, replicon containing S6.1-6 cells were implanted subcutaneously into previously irradiated SCID mice on day 1. All mice were imaged for the firefly luciferase bioluminescence following administration of the luciferin substrate. The mice were randomized and divided into treated and vehicle groups. The mice in the treated and vehicle groups had initial mean firefly luciferase bioluminescence within 10% of each other. Mice were treated with IFN or vehicle after imaging. On days 2 and 3 mice were imaged without any substrate to ensure that all luciferin bioluminescence had dissipated. Then, all mice were imaged for the renilla luciferase bioluminescence after administration of coelenterazine substrate. Four to five hours later, all mice were imaged without any substrate to ensure that all the renilla bioluminescence had dissipated. All mice were then imaged for the luciferase bioluminescence after administration of the luciferin substrate. Three naïve mice that had no cells implanted were imaged with coelenterazine to determine the background bioluminescence. The mean background signal was subtracted from the renilla bioluminescence of each of the mice to give the final renilla bioluminescence.

Results were as follows:

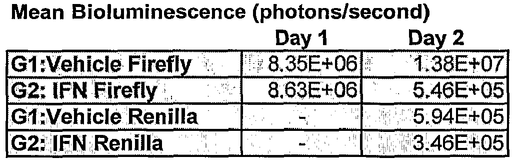

On day 2, the IFN-alpha treated group had a 25.2-fold decrease in firefly luciferase as compared to the vehicle group, whereas there was only a 1.7 decrease in renilla luciferase observed between the IFN-alpha treated and control groups. The significant decrease in the firefly signal but not the renilla signal is due to the specific effect of IFN-alpha upon the replicon. The data indicates there was no significant cell killing or toxicity in the IFN-alpha treated group versus the vehicle group that would account for the reduction in bioluminescence.

### Example 13: Treatment of subcutaneous model with or without test compound (eg IFN-ά.': Determination of Virus or Virus like particles in the blood

One example of a genomic replicon having a reporter gene has been described in Wakita et al., NATURE Medicine (2005) Vol. 11: pgs 791-796. The genomic replicon contains in 5' to 3' order the following elements: a 5' HCV UTR(including an HCV IRES); a luciferase gene; an EMCV IRES; remainder of the HCV genome (core protein through 3'UTR).

RNA from the genomic replicon is synthesized and used to transfect S6.1 cells.

Female SCID mice are gamma-irradiated at 3 GY for approximately 3.2 minutes on the day of and prior to cell implantation. 5x10⁶ cells containing the genomic replicon in 0.2 ml of vehicle are implanted subcutaneously into the right flanks of 42 mice, divided into three groups (control group, a low IFN-alpha dose group and a high IFN-alpha dose group). Each group contain 10 to 14 mice. Doses are given to each group as follows:
Control group: Vehicle (0.1 ml of 100 µµg/ml human serum albumin in HBSS) per day SC
Low IFN-alpha: 2,400 IU per day per 18 gram mouse IFN-alpha SC
High IFN-alpha: 200,000 IU per day per 18 gram mouse IFN-alpha SC

Interferon-alpha injection solution INTRON (Schering) is used. 18 million international units (IU) per vial; 3 million IU per 0.5 ml (NDC 0085-1168-01) Human Serum Albumin at 100ug/ml (Cat# A5843, Sigma, low endotoxin) are used as a carrier for the dilute IFN solutions. Dosing solutions are made fresh daily except weekends.

Just prior to dosing and twenty four hours after each dosing with IFN- alpha, blood is drawn from the animals and serum is prepared. The serum is directly incubated with a substrate for luciferase using a standard luciferase assay kit. Alternatively, the virus or virus like particle is subjected to centrifugation and pelleted. The pellet is incubated with lysis buffer and luciferase is assayed using a standard luciferase assay kit. A kit includes a lysis buffer and a substrate for luciferase. Relative Light Units (RLU) are determined and the signal obtained from IFN-alpha treated animals are compared to animals given the vehicle only. From these measurements, the degree of inhibition of virus or virus-like particles is determined. Similarly the same experrment can be performed after cells containing the replicon are introduced into the liver of mice as described in Example 11.

### Example 14: Treatment of Liver Model with Interferon-alpha

S6.1 cells are transfected with genomic replicon as described above in Example 13. The cells are harvested and re-suspended in HBSS at 1x10^6 cells/0.05 mls/injection. Female C.B17 SCID mice are shaved, gamma-irradiated at 3 GY for approximately 3.2 minutes. Mice are irradiated approximately 4 hours prior to surgery.

An incision is made through the skin and the peritoneal cavity, and then one liver lobe exposed gently. Tumor cells in 0.05 mls of HBSS are injected into the lobe at two sites (1x10^6 cells/0.05 mls/injection). The surgical incision is closed in the mice are allowed to recover from surgery overnight.

Mice are then randomized and either interferon-alpha or placebo is administered subcutaneously once a day beginning on day 1 and ending on day 7. Each group contains nine mice and the treatments are:
Group 1: Vehicle (0.1 ml of 100 ug/ml Human Serum Albumin in HBSS) SC, QD
Group 2: 200,000 IU per day per 18 gram mouse IFN-alpha 2b SC

Interferon-alpha injection solution INTRON (Schering) is used. 18 million international units (IU) per vial; 3 million IU per 0.5 ml (NDC 0085-1168-01)

Dosing solutions are made fresh daily except weekends.

Just prior to dosing and twenty-four hours after each dosing with IFN- alpha, blood is drawn from the animals and serum is prepared. The serum is directly incubated with a substrate for luciferase using a standard luciferase assay kit. Alternatively, the virus or virus like particle is subjected to centrifugation and pelleted. The pellet is incubated with lysis buffer and luciferase is assayed using a standard luciferase assay kit. A kit includes a lysis buffer and a substrate for luciferase. Relative Light Units (RLU) are determined and the signal obtained from IFN-alpha treated animals are compared to animals given the vehicle only. From these measurements, the degree of inhibition of virus or virus-like particles is determined.

### Example 15: In vivo selection of long term, stable mouse adapted replicon-containing cell clones.

*T7-11 cells.* Five million S6.1-6 cells in 0.2 ml of Hank's Balanced Salt Solution (HBSS) were implanted subcutaneously in female C.B17 SCID mice, which were irradiated (3 Gy) before cell implantation. A tumor emitting strong bioluminescence was excised 27 days later and the bioluminescent portion of the tumor was cultured *in vitro* in the presence of 0.5 mg/ml G418. Luciferase expression of G418-resistant colonies was evaluated using the Xenogen IVIS^{™} imaging system (Xenogen Corporation, Alameda, CA) and one colony with high expression was expanded to create the cell line T7-11. The selected colonies were expanded and implanted subcutaneously in gamma-irradiated SCID mice to evaluate the longevity of reporter *in vivo.* Most of the selected cell clones showed a decrease in signal over time *in vivo* similar to that seen in S6.1-6 cells. In contrast, the signal in one clone, T7-11, continued to increase to more than two logs over background two weeks after implantation as shown in **figure 12a** **and** **figure 15****.** This two log dynamic range was stable in T7-11 cells for more than two weeks.

To evaluate the stability of T7-11 cells in liver, which is the primary target organ for HCV infection, cells were directly injected into one lobe of the liver and bioluminescence signal was analyzed over time. T7-11 cells exhibited bioluminescence signal between one to two logs above background for more than one week, thus demonstrating the utility of this cell line for both SC and liver models. This data is shown in Figure 13d as control animals.

*Cell implantation.* Female SCID mice were shaved at the site of cell implantation and gamma-irradiated with 3 Gy either one-day prior to or on the same day as cell implant. For subcutaneous implantation, five million cells in 0.2 ml of HBSS were implanted at the right flank. For direct intra-hepatic implant, mice were anesthetized and one liver lobe was exteriorized through a small incision. One to two million cells in 20-50 µl of HBSS were injected directly into the liver parenchyma.

**L10-16 cells.** The process used to generate the T7-11 cell line was repeated using T7-11 cells as starter cells in place of S6.1 cells. Two million T7-11 cells were implanted into the livers of gamma irradiated female C.B17 SCID mice by direct hepatic injection. Thirty-seven days later, one mouse with a tumor with bioluminescence of 4.6x10^6 photons/second was euthanized and the tumor harvested from the liver and cultured *in vitro.* Colonies expressing G418 were selected in media containing 0.5 mg/ml Gentamycin and from these colonies luciferase positive clones were harvested. One of these G418 and luciferase positive clones was expanded to create the cell line L10-16.

L10-16 cells were implanted subcutaneously into gamma-irradiated SCID mice as was done for T7-11 . The mice showed stable expression of luciferase for more than one month. Results are shown in figure 15.

### Luciferase signal reflects RNA replication in T7-11 tumors

To rule out the possibility that the luciferase signal was not associated with HCV RNA replication, the viral RNA and protein level in the tumors were examined by northern blot probed with ³²P labeled negative stranded in vitro transcribed viral RNA and immunofluorescence assay (IFA) using an antibody against NS5B. The result showed that positive-strand HCV RNA was detected in T7-11 tumor tissue harvested 26 days after implantation (**Fig. 12b** lane 4). Consistent with this result, more than 20% of cells expressed viral protein NS5B. Referring to Figure 13c, immunoassay data with anti NS5Bfluorescent antibody indicates expression of NS5B in these cells. The data indicated that T7-11 cells supported constitutive HCV RNA replication and protein expression in vivo.

### Example 16: Efficacy of IFN-α 2B in γ-irradiated SCID mice subcutaneously implanted with T7-11 cells.

Efficacy studies with T7-11 implanted mice were initiated when the bioluminescence signal was stable and approximately two logs above background. Mice were treated with either 7500 IU/mouse/day or 15000IU/mouse/day IFN-α 2B (IFN) starting 19 days after implantation. Control animals received human albumin. As shown in **figure 13a****,** the mean bioluminescence signal in the low dose group (7,500IU/mouse/day) was reduced 5- and 10- fold after three and seven days of treatment, respectively, compared to the control group (P<0.01). A 10- and 25- fold reduction of signal was observed for the high dose group after three and seven days, respectively (P<0.001). The data clearly demonstrated that IFN-α 2B treatment had a dose-dependent effect on HCV RNA level. Moreover, Interleukin 2 (IL-2), which is known to activate the proliferation and function of T and natural killer (NK) cells *in vivo,* was used as a control to assess non-specific activity of IFN-α, a compound that may play a role in anti-tumor and immune-modulating response. Mice were treated with 1mg/kg of interleukin 2 (IL-2) based on the dose used for anti-tumor activity in renal cell cancer (RCC) models. Fan, K. et al. J. Immunol (2005) 175:7003-8). The result showed that IL-2 had no effect on bioluminescence signal over 5 days of treatment.

To demonstrate that the decrease in bioluminescence signal was directly related to compound exposure, dosing was ceased after three days of treatment. Rebound in viral RNA was clearly observed in the treated group 4 days after IFN-α 2B (15,000IU/mouse) treatment was discontinued, and the mean bioluminescence signal returned to the level that was similar to untreated group (P>0.05) (**Fig. 13b**). In contrast, the mean bioluminescence signal continued to decrease to background in the group treated with ongoing daily dosing (P<0.01) (**Fig. 13b**). Tumor tissues were excised for biochemical and immunohistochemical analysis at the end of the study to demonstrate that the HCV RNA levels were consistent with the bioluminescence levels. An average of more than 20% of cells exhibited detectable viral NS5B protein in control and rebound groups by IFA whereas less than 1% of cells stained positive for viral replication in IFN-α 2B treated group (**Fig. 13c****)**. The level of HCV RNA as measured by RT-PCR were consistent with the IFA data and indicated that RNA level in the tumor tissue from untreated mice was 10-fold higher than treated mice (data not shown). These results were consistent with the *in vivo* imaging data, indicating that bioluminescence signal accurately reflected the effect of IFN-α 2B on HCV RNA replication *in vivo.*

Although it has been reported that treatment of Huh 7 cells with IFN-α *in vitro* does not induce apoptosis, we could not rule out the possibility that IFN-α might trigger apoptotic programs leading to the elimination of virus containing tumor cells *in vivo.* To examine if cell death contributed to the decline of bioluminescence signal in treated mice, the fraction of apoptotic cells in tumor tissue was determined by immunohistochemical analysis of activated caspase 3 and cleaved poly (ADP-ribose) polymerase (cPARP). The results showed that both activated caspase 3 (data not shown) and cPARP staining were similarly present in 1-5% of tumor cells in all three groups (**Fig. 13c**). Staining with an antibody against Ki67, a marker of cell proliferation (data not shown), indicated that there also was no difference between the IFN-α 2B treated group and control group, suggesting that the reduction of bioluminescence signal observed in this model after treatment resulted from direct inhibition of viral replication.

Taken together, IFN-α 2B efficacy studies in the mouse model showed bioluminescence signal decreased in a dose-dependent manner in the treated group, which correlated to the decline of viral RNA and protein level in the tumor tissue. Reduction in bioluminescence signal was specifically due to the inhibition effect of IFN-α on replication, not cell death.

### Example 17: Efficacy of IFN-α 2B in liver model with T7-11 cells

To evaluate the efficacy of IFN-α in liver, T7-11 cells were directly injected into one lobe of the liver in gamma irradiated SCID mice. IFN-α 2B treatment at 15,000IU/mouse/day was initiated 27 days post inoculation when the signal was stable. Bioluminescence signal decreased more than 10-fold on the day2 of dosing in the IFN-α 2B treated group as compared to vehicle group (**Fig. 13d**) (P<0.0001). This result was very similar to that observed with the SC model (see **Fig. 13a**). Since the signal approached baseline after treatment, only a slight rebound was observed five days after removal of treatment (**Fig. 13d****).** To confirm that the luciferase signal was associated with the liver, the locations of tumors were determined by physical examination at the end of study

### Example 18: Effect of a small molecule, viral specific protease inhibitor (BILN 2061) on HCV in mouse models

The feasibility of using the models to evaluate the efficacy of a clinically tested, specific inhibitor of the HCV NS3/4A protease (BILN 2061) was examined. BILN 2061 was delivered through the subcutaneous route based on pharmacokinetic (PK) data that indicated that BILN 2061 displayed sustained plasma exposure for more than 24 hr. BILN 2061 was administered at 30mg/kg once daily starting day 21-post subcutaneous implantation of T7-11 cells. The results shown in figure 14a indicated that the bioluminescence signal decreased approximately two logs within three days compared to the control group (P=0.0006). As expected, several days after treatment was terminated, the signal returned to the levels similar to the untreated control group (P=0.5338). The data demonstrated that the compound effectively reduced the HCV RNA *in vivo* and did not have any adverse effect on the replicon-containing cells.

### Example 19: Efficacy of combination therapy with protease inhibitor (BILN 2061) and IFN-α in mouse models

The effect of protease inhibitor BILN 2061 on HCV replication by a combination of BILN 2061 and IFN-α 2B was determined. BILN 2061 is an NS3 protease as described previously (Lamarre, D. et al. An NS3 protease inhibitor with antiviral effects in humans infected with hepatitis C virus. Nature 426, 186-9 (2003). The treatment was initiated on day 18 after subcutaneous implantation of T7-11 cells in gamma irradiated SCID mice. Three groups of mice were administrated SC daily with 30mg/kg of BILN 2061 alone, 15,000IU/ml of IFN-α alone or a combination of two agents, respectively. Two days after treatment, the mean bioluminescence signal decreased 16-fold or 7-fold in mice treated with either BILN 2061 or IFN-α 2B as compared to the control group (P<0.01) (**Fig. 14b**). In contrast, approximately 100-fold reduction in bioluminescence was observed in mice treated with the BILN 2061/IFN-α 2B combination therapy (P<0.001) (**Fig. 14b**). Clearly the combination therapy inhibited HCV RNA replication more efficiently than treatment with either single agent alone, demonstrating the utility of this model for evaluating new therapeutic strategies for HCV treatment.

### Example 20: Selection of cells with decreased sensitivity to human IFN--α 2B

Preliminary data showed that RNA replication in T7-11 cells was five to eight fold more resistant to human IFN-α 2B *in vitro* than that in parental S6.1-6 cells, suggesting that the antiviral responses in the T7-11 cells appeared to be attenuated, a condition that would account for the observed increased stability of replicon RNA *in vivo.* We also found that this IFN-α insensitive phenotype was associated with adaptive mutations acquired at the cellular level, not at the viral RNA level.

T7-11 cells or L10-16 cells are maintained the IFN insensitive phenotype as determined by EC₅₀ of IFN-α *in* vitro. These cells can be cured of replicon following continuous culturing in high does IFN-α. The cells are grown to confluence and the media is supplemented with 200 IU/ml IFN-a. The cells are maintained under these conditions for two weeks. After two weeks, the cells are split and grown in complete media either with or without G418. Lack of growth in media containing G418 indicates that the replicon is no longer present in the cells. The resulting cells lines are referred to as "T7-11C" and "L10-6C" (cured) Upon transient transfection of an identical replicon RNA (rep114/ET) into S6.1, cured T7-11 cells ("T7-11C" in Figure 17) and cured L10-16 cells ("L10-16C" in Figure 17), all of which were cleared of replicon RNA by IFN-α treatment *in vitro,* only cured T7-11 cells maintained the IFN insensitive phenotype as determined by EC₅₀ of IFN-α *in vitro.* Upon transient transfection of an identical replicon RNA (rep114/ET) into S6.1, cured T7-11 cells ("T7-11C" in Figure 17) and cured L10-16 cells ("L10-16C" in Figure 17), all of which were cleared of replicon RNA by IFN-α treatment *in vitro,* only cured T7-11 cells maintained the IFN insensitive phenotype as determined by EC₅₀ of IFN-α *in vitro.*

The above methods can be used to isolate tumor cell lines with decreased snensitivity to other immunomodulatory compounds in addition to interferon alpha.

### Example 21: Effect of protease inhibitor on in vivo replication of T7-11 cells

The antiviral effect of BILN 2061 on HCV replication was also examined. BILN 2061 was administered subcutaneously at 30mg/kg once daily starting day 25-post tumor cell implantation. A 10-fold decline in bioluminescence within three days was observed in the treated group compared to the untreated control group (P<0.0001) (**Fig. 16**). Taken together, the effect of IFN-α and BILN 2061 in the liver model was very similar to that observed in the subcutaneous model (see **Fig. 13b** & **13d**), demonstrating the utility of both models for evaluation of anti-HCV compounds.

In additional to the specific examples, the following general methods were employed:

### Methods

**Cell cultures** Human hepatoma cell line Huh-7 (Cancer Res 42:3858-63) was maintained in Dulbecco's modified Eagle medium (DMEM) (Invitrogen, Carlsbad, CA) containing high glucose, 10% fetal bovine serum, L-glutamine, and non-essential amino acids, at 37⁰C in 5% CO₂. G418 (Geneticin) (Invitrogen, Carlsbad, CA) was added at a final concentration of 0.5mg/ml for replicon-containing cell clones. **Plasmid Construction** The replicon rep114/ET was constructed by replacing the 1.6kb XbaI-NotI fragment from pFKI341PILucNS3-3'/ET with the 2.6kb XbaI-NotI fragment from pFKI389LucUbiNeoNS3-3'/5.1.

**RNA preparation and electroporation** Plasmids were linearized with *Sca*I, and RNA was synthesized with the MEGAscript kit (Ambion, Austin, TX). RNA was extracted and purified using TRIzol following manufacturer's instructions (Invitrogen, Carlsbad, CA). RNA transfection was performed as described previously.

### Subcloning S6.1, S6.1-6 and T7-11 cell lines

*S6.1 cells*: Five million Huh 5-2 cells were implanted subcutaneously into the right flank of 10 female SCID/bg mice. The three largest tumors were harvested 37 days post implantation of cells and mashed into brei with the frosted ends of two sterilized glass slides. 50 - 100 µl of brei was inserted subcutaneously with a trocar into each of 10 anesthetized SCID/bg mice. This *in vivo* passaging process was repeated two more times with brei from tumors harvested on days 22 and 28, respectively. Twenty-four days post final implantation, three of the larger tumors were harvested and tumor cells were expanded in cell culture to create the cell line S6.1.

*S6.1-6 cells.* 5µg of *in vitro*-transcribed rep114/ET RNA was electroporated into S6.1 cells. After electroporation, stable cell clones were selected in medium containing 0.5mg/ml of G418. Luciferase expression of G418 resistant colonies was evaluated²⁰ and the colony with highest expression was expanded to create the cell line S6.1-6. *T7-11 cells.* Five million S6.1-6 cells in 0.2 ml of Hank's Balanced Salt Solution (HBSS) were implanted subcutaneously in female C.B17 SCID mice, which were irradiated (3 Gy) before cell implantation. A tumor emitting strong bioluminescence was excised 27 days later and the bioluminescent portion of the tumor was cultured *in vitro* in the presence of 0.5 mg/ml G418. Luciferase expression of G418-resistant colonies was evaluated using the Xenogen IVIS^{™} imaging system (Xenogen Corporation, Alameda, CA) and one colony with high expression was expanded to create the cell line T7-11.

**Analysis of HCV RNA by Northern hybridization** Total RNA was extracted from tumor tissue samples with TRIzol reagent (Invitrogen, Carlsbad, CA). Ten micrograms of total RNA was fractionated on a 1% agarose gel containing 2.2M formaldehyde and transferred onto a nylon membrane (Ambion, Austin, TX), which was hybridized with a ³²P-labeld negative-sense Riboprobe (Promega, Madison, WI) complementary to the 3'end of NS5A and 5' end of NS5B.

**Immunohistochemical analysis.** Four-micron thick sections were prepared from either formalin-fixed or 4% paraformaldehyde-fixed, paraffin-embedded tumors. For detection of cleaved PARP and activated caspase 3, a polyclonal rabbit anti-cleaved PARP antibody (BioSource, Camarillo CA) and a polyclonal rabbit anti-cleaved caspase 3 antibody (CalBiochem, San Diego, CA) were employed along with biotinylated anti-rabbit secondary antibody (Jackson Labs, West Grove, PA). For Ki-67, a mouse monoclonal anti-human Ki-67 antibody (Ventana Medical Systems, Tucson AZ) and a biotinylated anti-mouse IgG1 secondary antibody (Research Diagnostics Inc., Flanders, NJ) were used. For detection of viral protein NS5B expression, a polyclonal rabbit anti-NS5B antibody (Chiron Corporation, Emeryville, CA)³⁶ and a fluorescence isothiocyanate (FITC)-conjugated goat anti-rabbit immunoglobulin antibody (Invitrogen, Carlsbad, CA) were used.

### In Vivo Studies

Female C.B17 SCID (SCID), SCID/bg, Nu/Nu and Balb/c mice (5-13 weeks old) were obtained from Charles River Laboratories (Wilmington, MA). All studies were conducted in an AALAS certified facility under the direction of the Institutional Animal Care and Use Committee.

*Cell implantation.* Female SCID mice were shaved at the site of cell implantation and gamma-irradiated with 3 Gy either one-day prior to or on the same day as cell implant. For subcutaneous implantation, five million cells in 0.2 ml of HBSS were implanted at the right flank. For direct intra-hepatic implant, mice were anesthetized and one liver lobe was exteriorized through a small incision. One to two million cells in 20-50 µl of HBSS were injected directly into the liver parenchyma.

*Whole body* in vivo *imaging.* Anesthetized mice were injected intraperitoneally with 150 mg/kg of luciferin (Xenogen Corporation, Alameda, CA). After 10-20 minutes, mice were imaged using a CCD camera in the Xenogen IVIS^{™} imaging system. The bioluminescence signal intensity was quantified using Living Image Software (Xenogen Corporation, Alameda, CA).

*Drug Treatment.* IFN-α 2b (Intron^{®} A; Schering-Plough, NJ), BILN 2061 and rIL-2 (Aldesleukin/Proleukin^{®}) (Chiron Corporation, Emeryville, CA) were injected subcutaneously in the supra scapular area.

*Statistical analysis.* Differences in bioluminescence signal between control and treatment groups ofmice were compared by the two-tailed Mann-Whitney test. Differences in signal among the control, treatment and rebound groups ofmice and among combination groups were compared by the nonparametric Kmskal-Wallis test A P-value < 0.05 was considered significant.

Although the foregoing invention has been described in some detail for purposes of clarity of understanding, it will be apparent that certain changes and modifications may be practiced within the scope of the invention. It should be noted that there are many alternative ways of implementing both the process and compositions of the present invention. Accordingly, the present embodiments are to be considered as illustrative and not restrictive, and the invention is not to be limited to the details given herein.

All references cited are incorporated herein by reference in their entirety and for all purposes.

### REFERENCES

[1] Wu et al. (2004) Antiviral Res. 64(2):79-83.
[2] Vasilakos et al. (2000) Cell Immullol. 204(1):64-74.
[3] US patents 4689338, 4929624, 5238944, 5266575, 5268376, 5346905, 5352784, 5389640, 5395937, 5482936, 5494916, 5525612, 6083505, 6440992, 6627640, 6656938, 6660735, 6660747, 6664260, 6664264, 6664265, 6667312; 6670372, 6677347, 6677348, 6677349, 6683088, 6703402, 6743920, 6800624, 6809203, 6888000 and 6924293.
[4] US 6,924,271.
[5] US2005/0070556.
[6] US 5,658,731.
[7] WO02/18383.

### Numbered Embodiments

1. An animal useful for determining the replication, expression or production of HCV virus or virus-like particles from an HCV replicon, the animal comprising:
   a cell containing an HCV replicon comprising a reporter gene;
   wherein the animal is immunocompromised.
2. The animal of embodiment 1 wherein the cell containing the HCV replicon is selected from the group consisting of: primary hepatocytes; stem cells; kidney cells; liver cells; bone marrow-derived cells; hepatomas; hepatoblastomas.
3. The animal of embodiment 1 or 2, wherein the cell containing the replicon is a human cell or is derived from a human cell.
4. The animal of embodiment 1, wherein the cell containing the HCV replicon is an immortalized cell.
5. The animal of any of embodiments 1-4, wherein the cell containing the replicon is a tumor cell.
6. The animal of any of embodiment 5, wherein the cell is selected from Huh 7 cells, HeLa cells. HEK293, IMY cells, FLC4 cells, and HepG2 cells.
7. The animal of embodiment 6, wherein the cell is derived from an Huh 7 cell.
8. The animal of embodiment 7, wherein the Huh 7 derived cell is an Huh 5-2 cell.
9. The animal of any of embodiments 1-8, wherein the animal is a small animal.
10. The animal of embodiment 9, wherein the small animal is a rodent.
11. The animal of embodiment 10, wherein the rodent is murine.
12. The animal of any of embodiments 1-11, wherein the animal is a non-transgenic animal.
13. The animal of any of embodiments 1-12, wherein the cells containing the HCV replicon are adapted to growth in the animal by a plurality of passages through the organism.
14. The animal of any of embodiments 1-13, wherein the cell containing the replicon is adapted to have improved growth kinetics in the animal.
15. The animal of any of embodiments 1-14, wherein the cell containing the HCV replicon is adapted to have improved replicon stability in the animal.
16. The animal of any of embodiments 13-15, wherein the adapted cell has been serially subcutaneously passaged *in vivo* in the animal.
17. The animal of any of embodiments 1-16, wherein the HCV replicon is a sub-genomic replicon.
18. The animal of any of embodiments 1-16, wherein the HCV replicon is a genomic replicon.
19. The animal of any of embodiments 1-18, wherein the reporter gene is part of a monocistronic RNA encoding the HCV polyprotein or a fragment thereof.
20. The animal of embodiment 19, wherein the reporter gene is cleaved from the HCV protein(s) by HCV protease.
21. The animal of embodiment 19, wherein the reporter gene product remains fused to an HCV protein following replication and expression of the HCV replicon and cleavage of the HCV polyprotein.
22. The animal of embodiment 21, wherein the reporter gene product remains fused to an HCV structural protein.
23. The animal of embodiment 21, wherein the reporter gene product remains fused to an HCV non-structural protein.
24. The animal of embodiment 23, wherein the reporter gene product remains fused to NS5A.
25. The animal of any of embodiments 1-24, wherein the reporter gene encodes a product selected from the group consisting of: an enzyme; and an antigen detectable with a known antibody and a protein that fluoresces.
26. The animal of embodiment 25, wherein the reporter gene encodes an enzyme that is a luciferase.
27. The animal of any of embodiments 1-26, wherein the cell containing the HCV replicon further expresses at least one additional gene.
28. The animal of embodiment 27, wherein the additional gene(s) include a second reporter gene.
29. The animal of embodiment 28, wherein the second reporter gene is a marker for viability of cells introduced into the animal.
30. The animal of any of embodiments 27-28, wherein the additional gene(s) include a caveolin.
31. The animal of embodiment 30, wherein the caveolin is caveolin-3 or a functional equivalent of caveolin-3.
32. The animal of embodiment 31, wherein the caveolin-3 is human caveolin-3.
33. The animal of any of embodiments 27-32, wherein one or more of the additional gene(s) is encoded in a plasmid.
34. The animal of any of embodiments 27-33, wherein one or more of the additional gene(s) is encoded in the HCV replicon.
35. The animal of any of embodiments 27-34, wherein one or more of the additional gene(s) is integrated in the genome of the cell containing the HCV replicon.
36. The animal of any of embodiments 27-35, wherein one or more of the additional genes is heterologous to HCV, the cells containing the HCV replicon and the animal.
37. The animal of any of embodiments 1-36,wherein the animal is immuno-compromised by at least one of: genetic mutation, irradiation and chemical immuno-suppression.
38. The animal of embodiment 37, wherein the animal is gamma-irradiated.
39. The animal of any of embodiments 1-38, wherein the animal is a SCID animal.
40. The animal of any of embodiments 1-39, wherein replicon expression and/or replication of the HCV replicon is maintained for at least two days after the cell containing the replicon is introduced into the animal.
41. The animal of embodiment 40, wherein replicon expression and/or replication of the HCV replicon is maintained for at least five days after the cell containing the replicon is introduced into the animal.
42. The animal of embodiment 40, wherein replicon expression and/or replication of the HCV replicon is maintained for at least seven days after the cell containing the replicon is introduced into the animal.
43. The animal of any of embodiments 1-42, wherein the cell containing the HCV replicon are introduced into the animal by injection of the cell containing the HCV replicon into the portal vein of the animal.
44. The animal of any of embodiments 1-42, wherein the cell containing the HCV replicon is implanted into the liver of the animal.
45. The animal of any of embodiments 1-42, wherein the cells containing the HCV replicon are implanted subcutaneously into the animal.
46. A method of identifying a compound that reduces or inhibits HCV replication comprising:
   a) providing an immuno-compromised animal according to any one of embodiments 1 to 45;
   b) administering a candidate compound to the animal; and
   c) determining the level of activity of the reporter gene contained within the replicon in the animal as compared to the level of activity of the reporter gene contained within the replicon in an animal as provided in step a) that is not contacted with the compound;
      wherein a compound that reduces or inhibits the level of activity of the reporter gene contained within the replicon in the animal contacted with the compound is a compound that inhibits or reduces HCV replication.
47. The method of embodiment 46, wherein the course of replication is observed for at least two days after introduction of a cell containing the HCV replicon into the animal.
48. The method of embodiment 46, wherein the course of replication is observed for at least five days after introduction of a cell containing the HCV replicon into the animal.
49. The method of embodiment 46, wherein the course of replication is observed for at least seven days after introduction of the cells containing the HCV replicon into the animal.
50. The method of any of embodiments 46-49, wherein the animal is immunocompromised before the cells containing the HCV replicon are introduced into the animal.
51. The method of any of embodiments 46-49, wherein the animal is immunocompromised after the cells containing the HCV replicon are introduced into the animal.
52. The method of any of embodiments 46-49, wherein the animal is immunocompromised at the same time that the cells containing the HCV replicon are introduced into the animal.
53. The method of any of embodiments 46-52, wherein the cells are introduced into the animal by subcutaneous implantation.
54. The method of any of embodiments 46-52, wherein the cells are implanted into the liver of the animal.
55. The method of any of embodiments 46-52 wherein the cells are introduced into the animal via the portal vein and then migrate to the liver of the animal.
56. The method of any of embodiments 46-55, wherein the animal is immuno-compromised by gamma-irradiation.
57. A method of identifying a compound that reduces or inhibits HCV particle production comprising:
   a) providing an immuno-compromised animal according to any one of embodiments 1 to 43;
   b) administering a compound to the animal; wherein the compound does not reduce or inhibit replicon replication or expression and
   c) determining the level of activity of the reporter gene contained within the replicon in the animal as compared to the level of activity of the reporter gene contained within the replicon in an animal as provided in step a) that is not contacted with the compound;
      wherein a compound that reduces or inhibits the level of activity of the reporter gene contained within the replicon in the animal contacted with the compound is a compound that inhibits or reduces HCV particle production.
58. The method of embodiment 57, wherein the course of replication is observed for at least two days after introduction of a cell containing the HCV replicon into the animal.
59. The method of embodiment 58, wherein the course of replication is observed for at least five days after introduction of a cell containing the HCV replicon into the animal.
60. The method of embodiment 59, wherein the course of replication is observed for at least seven days after introduction of the cells containing the HCV replicon into the animal.
61. The method of any of embodiments 57-60, wherein the animal is immunocompromised before the cells containing the HCV replicon are introduced into the animal.
62. The method of any of embodiments 57-60, wherein the animal is immunocompromised after the cells containing the HCV replicon are introduced into the animal.
63. The method of any of embodiments 57-60, wherein the animal is immunocompromised at the same time that the cells containing the HCV replicon are introduced into the animal.
64. The method of any of embodiments 57-60, wherein the cells are introduced into the animal by subcutaneous implantation.
65. The method of any of embodiments 57-60, wherein the cells are implanted into the liver of the animal.
66. The method of any of embodiments 57-60 wherein the cells are introduced into the animal via the portal vein and then migrate to the liver of the animal.
67. The method of any of embodiments 57-66, wherein the animal is immuno-compromised by gamma-irradiation.
68. The method of any of embodiments 46 to 67 wherein a compound which reduces or inhibits HCV replication or particle formation is administered to said animal prior to administering a candidate compound to said animal.
69. A pharmaceutical composition comprising a therapeutically effective amount of a first antiviral agent and a therapeutically effective amount of a second antiviral agent.
70. The pharmaceutical composition of embodiment 69 wherein the first antiviral agent is an interferon or an HCV protease inhibitor.
71. The pharmaceutical composition of embodiment 70 wherein said first antiviral agent is an interferon.
72. The pharmaceutical composition of embodiment 71 wherein said interferon is an α-interferon or pegylated α-interferon.
73. The pharmaceutical composition of embodiment 71 wherein said second antiviral agent is an NS3/4A protease inhibitor.
74. The pharmaceutical composition of embodiment 73 wherein said NS3/4A protease inhibitor is BILN 2061.
75. The pharmaceutical composition according to embodiments 69 , wherein said second antiviral agent is selected from immunomodulatory agents, inhibitors of HCV helicase, inhibitors of HCV polymerase or inhibitors of an HCV protease, ribavirin, amantadine, VX-497 (merimepodib, Vertex Pharmaceuticals), VX-498 (Vertex Pharmaceuticals), Levovirin, Viramidine, Ceplene (maxamine), XTL-001 and XTL-002 (XTL Biopharmaceuticals) ANA975 (Anadys), MN283-Valopictitabine, Idenix).
76. The pharmaceutical composition according to any of embodiments 75, wherein said second antiviral agent is an anti-HCV antiviral agent.
77. The pharmaceutical composition of embodiment 76 wherein said anti-HCV antiviral agent is selected from the group consisting of inhibitors of HCV helicase, inhibitors of HCV polymerase or inhibitors of an HCV protease, ribavirin, amantadine, VX-497 (merimepodib, Vertex Pharmaceuticals), VX-498 (Vertex Pharmaceuticals), Levovirin, Viramidine, Ceplene (maxamine), XTL-001 and XTL-002 (XTL Biopharmaceuticals) ANA975 (Anadys), MN283-Valopictitabine, Idenix), siRNA and antisense RNA.
78. The pharmaceutical composition of embodiment 69 or 70 wherein one of said anti-viral agents is selected from the group consisting of imidazoquinolines, SMIPS and CpG oligonucleotides.
79. A method for the treatment or prevention of a HCV infection in a mammal by administering to the mammal an anti-HCV effective amount of the pharmaceutical composition of any of embodiments 69-78.
80. The method of embodiment 79 wherein the compounds are administered sequentially.
81. The method of embodiment 80 wherein the compounds are administered simultaneously.
82. A method for establishing a cell line as described in any of the General Methods described herein and the specific Examples.
83. A cell line as described in any of the General Methods described herein and the specific Examples.
84. A cell line as in embodiment 83 wherein the cell line is less sensitive to an immunomodulatory agent after passage through an animal of the invention as described herein.
85. A cell line as in embodiment 84 wherein the cell line is less sensitive to interferon alpha after passage through an animal of the invention as described herein.
86. A cell line having a decreased sensitivity to interferon-α derived by the following process:
   implanting said cells containing an HCV replicon comprising a reporter gene into an immunocompromised mouse;
   excising from the mouse after implantation a tumor emitting strong bio luminescence;
   culturing *in vitro* the bioluminescent portion of the tumor in the presence of a positive selecting agent for the cells;
   selecting a cell colony with high luciferase expression and culturing said cells
87. A cell line as in embodiment 86 that is a tumor cell line.
88. A tumor cell line as in embodiment 87 wherein the mouse is irradiated prior to implanting the tumor cells containing the replicon.
89. A tumor cell line as in embodiment 86 to 88 wherein the tumor is excised from the mouse between 2 and 4 weeks after implantation.
90. A tumor cell line as in embodiment 89 wherein the selecting agent is G418.
91. A tumor cell line as in embodiment 86-90 wherein the cells are continuously exposed to interferon alpha until the cells no longer contain the replicon.
92. A tumor cell line as in embodiment 91 wherein the cells are exposed to interferon alpha at a dose of about 200 IU/ml interferon alpha.
93. A tumor cell line selected from the group consisting of the tumor cell lines T7-11; T7-11C; L10-6; and L10-6C.
94. A tumor cell line as in any of embodiments 86 to 92 where the immunocompromised mouse is a SCID mouse.

## Claims

1. A pharmaceutical composition comprising a therapeutically effective amount of a first antiviral agent and a therapeutically effective amount of a second antiviral agent.

2. The pharmaceutical composition of claim 1 wherein the first antiviral agent is an interferon or an HCV protease inhibitor.

3. The pharmaceutical composition of claim 2 wherein said first antiviral agent is an interferon.

4. The pharmaceutical composition of claim 3 wherein said interferon is an α-interferon or pegylated α-interferon.

5. The pharmaceutical composition of claim 3 wherein said second antiviral agent is an NS3/4A protease inhibitor.

6. The pharmaceutical composition of claim 5 wherein said NS3/4A protease inhibitor is BILN 2061.

7. The pharmaceutical composition according to any one of claims 1-6 , wherein said second antiviral agent is selected from immunomodulatory agents, inhibitors of HCV helicase, inhibitors of HCV polymerase or inhibitors of an HCV protease, ribavirin, amantadine, VX-497 (merimepodib, Vertex Pharmaceuticals), VX-498 (Vertex Pharmaceuticals), Levovirin, Viramidine, Ceplene (maxamine), XTL-001 and XTL-002 (XTL Biopharmaceuticals) ANA975 (Anadys), MN283-Valopictitabine, Idenix).

8. The pharmaceutical composition according to any of claims 1-7, wherein said second antiviral agent is an anti-HCV antiviral agent.

9. The pharmaceutical composition of claim 8 wherein said anti-HCV antiviral agent is selected from the group consisting of inhibitors of HCV helicase, inhibitors of HCV polymerase or inhibitors of an HCV protease, ribavirin, amantadine, VX-497 (merimepodib, Vertex Pharmaceuticals), VX-498 (Vertex Pharmaceuticals), Levovirin, Viramidine, Ceplene (maxamine), XTL-001 and XTL-002 (XTL Biopharmaceuticals) ANA975 (Anadys), MN283-Valopictitabine, Idenix), siRNA and antisense RNA.

10. The pharmaceutical composition of claim 1 or 2 wherein one of said anti-viral agents is selected from the group consisting of imidazoquinolines, SMIPS and CpG oligonucleotides.

11. The pharmaceutical composition of any of claims 1-10 for use in a method of treating or preventing HCV infection in a mammal.

12. The pharmaceutical composition of claim 11 **characterized in that** the treating or preventing comprises administering the first and second antiviral agents sequentially.

13. The pharmaceutical composition of claim 11 **characterized in that** the treating or preventing comprises administering the first and second antiviral agents simultaneously.


SEQUENCE LISTING

<110>   Novartis AG

<120>   SMALL ANIMAL MODEL FOR HCV REPLICATION

<130>   P062028EP

<150>   EP06787397.6
<151>   2006-07-15

<150>   US60/700475
<151>   2005-07-18

<150>   US60/776640
<151>   2006-02-23

<160>   6

<170>   SeqWin99, version 1.02

<210>   1
<211>   151
<212>   PRT
<213>   Homo sapiens

<400>   1

<210>   2
<211>   20
<212>   DNA
<213>   Artificial Sequence

<220>
<223>   CpG oligonucleotide

<400>   2
tccatgacgt tcctgacgtt                                                   20

<210>   3
<211>   18
<212>   DNA
<213>   Artificial Sequence

<220>
<223>   CpG oligonucleotide

<400>   3
tctcccagcg tgcgccat                                                     18

<210>   4
<211>   30
<212>   DNA
<213>   Artificial Sequence

<220>
<223>   CpG oligonucleotide

<400>   4
accgatgacg tcgccggtga cggcaccacg                                        30

<210>   5
<211>   24
<212>   DNA
<213>   Artificial Sequence

<220>
<223>   CpG oligonucleotide

<400>   5
tcgtcgtttt gtcgttttgt cgtt                                              24

<210>   6
<211>   20
<212>   DNA
<213>   Artificial Sequence

<220>
<223>   CpG oligonucleotide

<400>   6
tccatgacgt tcctgatgct                                                   20